# EUROPÄISCHE PATENTSCHRIFT

(11) **EP 1 685 261 B1**
(45) Veröffentlichungstag und Bekanntmachung des Hinweises auf die Patenterteilung: **25.03.2015**
(21) Anmeldenummer: 04803181.9
(22) Anmeldetag: 18.11.2004
(51) Int. Cl.: C12Q 1/68, A61K 48/00

(54) **HOCHPARALLELER DNA-SYNTHESIZER AUF MATRIZENBASIS**
HIGHLY PARALLEL DNA SYNTHESISER BASED ON MATRICES
SYNTHETISEUR D'ADN A PARALLELISME ELEVE SUR UNE BASE MATRICIELLE

(30) Priorität: 18.11.2003 DE 10353887
(43) Veröffentlichungstag der Anmeldung: 02.08.2006
(73) Patentinhaber: Synthetic Genomics, Inc., La Jolla, CA 92037 (US)
(72) Erfinder: STÄHLER, Peer, 68167 Mannheim (DE); STÄHLER, Cord, 69469 Weinheim (DE); BEIER, Markus, 69121 Heidelberg (DE)
(74) Vertreter: Weiss, Wolfgang
(86) Internationale Anmeldenummer: PCT/EP2004/013131
(87) Internationale Veröffentlichungsnummer: WO 2005/051970

(56) Entgegenhaltungen:
- EP-A2- 1 180 548
- WO-A-00/49142
- WO-A-01/48242
- WO-A-93/17126
- WO-A-99/05321
- WO-A-99/32654
- US-A- 6 013 440
- WANG J ET AL: "DNA microarrays with unimolecular hairpin double-stranded DNA probes: Fabrication and exploration of sequence-specific DNA/protein interactions" JOURNAL OF BIOCHEMICAL AND BIOPHYSICAL METHODS, AMSTERDAM, NL, Bd. 55, Nr. 3, März 2003 (2003-03), Seiten 215-232, XP002251375 ISSN: 0165-022X
- BEIER M ET AL: "Analysis of DNA-microarrays produced by inverse in situ oligonucleotide synthesis" JOURNAL OF BIOTECHNOLOGY, ELSEVIER SCIENCE PUBLISHERS B.V., AMSTERDAM, NL, Bd. 94, Nr. 1, 14. März 2002 (2002-03-14), Seiten 15-22, XP002281008 ISSN: 0168-1656

## Beschreibung

### 1.1 Einleitung

Die Herstellung von synthetischen Nukleinsäuren (DNA, RNA oder deren Analoga) wird überwiegend mit Hilfe von säulengestützten Synthesizern durchgeführt. Der Bedarf an solchen synthetischen Nukleinsäuren ist durch die Molekularbiologie und die biomedizinische Forschung und Entwicklung stark gestiegen.

Besonders wichtige und weit verbreitete Einsatzgebiete für synthetische Nukleinsäurepolymere sind Primer für die *poymerase chain reaction* (PCR) [Critical Reviews in Biochemistry and Molecular Biology 26 (3/4), S. 301-334, 1991] und die Sequenziermethode nach Sanger [Proc. Nat. Acad. Sci. 74, S. 5463-5467, 1977].

Synthetische DNA spielt auch für die Herstellung von synthetischen Genen eine Rolle [1. WO 00/13017 A2, 2. S. Rayner et al., PCR Methods and Applications 8 (7), S. 741-747, 1998, 3. WO 90/00626 A1, 4. EP 385 410 A2, 5. WO 94/12632 A1, 6. WO 95/17413 A1, 7. EP 316 018 A2, 8. EP 022 242 A2, 9. L. E. Sindelar and J. M. Jaklevic, Nucl. Acids Res. 23 (6), S. 982-987, 1995, 10. D. A. Lashkari, Proc. Nat. Acad. Sci. USA 92 (17), S. 7912-7915, 1995, 11. WO 99/14318 A1].

Zwei neuere Anwendungsfelder mit steigendem Bedarf sind die Herstellung von Mikroarrays aus Oligonukleotid-Sonden [1. Nature Genetics, Vol. 21, supplement (gesamt), Jan. 1999, 2. Nature Biotechnology, Vol. 16, S. 981-983, Okt. 1998, 3. Trends in Biotechnology, Vol. 16, S. 301-306, Jul. 19989] und die Herstellung von interferrierender RNA (iRNA oder RNAi) für die Modulation der Genexpression in Zielzellen [PCT/EP01/13968].

Die genannten Anwendungsgebiete der Molekularbiologie liefern wertvolle Beiträge in der Wirkstoff-Entwicklung, der Wirkstoff-Produktion, der kombinatorischen Biosynthese (Antikörper, Effektoren wie Wachstumsfaktoren, Neurotransmitter etc.), in der Biotechnologie (z.B. Enzymdesign, Pharming, biologische Herstellungsverfahren, Bioreaktoren etc.), in der molekularen Medizin, in der Entwicklung und Anwendung von Diagnostika (Mikroarrays, Rezeptoren und Antikörper, Enzymdesign etc.) oder in der Umwelttechnik (spezialisierte oder maßgeschneiderte Mikroorganismen, Produktionsverfahren, Sanierung, Sensoren etc.). Die Anwendung des erfindungsgemäßen Verfahrens kann somit in allen diesen Bereichen erfolgen.

### 1.2 Stand der Technik

Die am weitesten verbreitete Methode zur Herstellung von synthetischen Nukleinsäuren basiert auf Grundlagenarbeiten von Caruthers und wird als Phosphitamid-Methode beschrieben (M. H. Caruthers, Methods in Enzymology 154, S. 287-313, 1987). Die Sequenz der entstehenden Moleküle kann dabei durch die Syntheseabfolge gesteuert werden. Andere Verfahren, wie z.B. die H-Phosphonat-Methode, dienen dem gleichen Zweck des sukzessiven Aufbaues eines Polymers aus seinen Untereinheiten, haben sich aber nicht so weit durchsetzen können wie die Methode nach Caruthers.

Um das chemische Verfahren der Polymersynthese aus Untereinheiten automatisieren zu können, werden meistens feste Phasen verwendet, an denen die wachsende Molekülkette verankert ist. Sie wird erst nach Fertigstellung der Synthese abgespalten, wozu ein geeigneter Linker zwischen dem eigentlichen Polymer und der festen Phase erforderlich ist. Die Methode verwendet zur Automatisierung in der Regel feste Phasen in Form aktivierter Partikel, die in eine Säule gefüllt werden, z.B. *controled pore glass* (CPG). Solche festen Phasen tragen in der Regel nur eine definiert trenn- und entnehmbare Art von Sequenz. Die Zugabe der einzelnen Synthesereagenzien erfolgt nun in einer steuerbaren Art und Weise in einem Automaten, der vor allem die automatisierte Zugabe der einzelnen Reagenzien zur festen Phase sicherstellt. Die Menge an synthetisierten Molekülen ist durch die Menge des Trägermaterials und die Größe der Reaktionsansätze steuerbar. Diese Mengen sind für die oben genannten molekular-biologischen Verfahren entweder ausreichend oder sogar zu hoch (z.B. bei PCR-Primern). Eine gewisse Parallelisierung zur Erzeugung einer Vielzahl unterschiedlicher Sequenzen wird durch Anordnung von mehreren Säulen in einem apparativen Aufbau erreicht. So sind dem Fachmann Geräte mit 96 parallelen Säulen bekannt.

Eine Variante und Weiterentwicklung der Herstellung von synthetischen Nukleinsäuren ist die *in situ* Synthese von Mikroarrays (Array-Anordnung der Nukleinsäuren in einer Matrix). Diese wird auf einem Substrat durchgeführt, das durch die Synthese mit einer Vielzahl unterschiedlicher Sequenzen beladen wird. Ein Ablösen der Syntheseprodukte ist dabei nicht vorgesehen. Der große Vorteil der *in situ* Syntheseverfahren für Mikroarrays ist die Bereitstellung einer Vielzahl von Molekülen unterschiedlicher und definierter Sequenz an adressierbaren Lokationen auf einem gemeinsamen Träger. Die Synthese greift dabei auf ein überschaubares Set aus Einsatzstoffen zurück (bei DNA-Mikroarrays in der Regel die 4 Basen A, G, T und C) und baut aus diesen beliebige Sequenzen der Nukleinsäurepolymere auf.

Die Abgrenzung der einzelnen Molekülspezies kann zum einen durch getrennte fluidische Kompartimente bei der Zugabe der Syntheseeinsatzstoffe erfolgen, wie es z.B. in der so genannten *in situ* Spotting Methode oder Piezoelektrischen Techniken der Fall ist, beruhend auf der Tintenstrahldrucktechnik (A. Blanchard, in Genetic Engineering, Principles and Methods, Vol. 20, Ed. J. Sedlow, S. 111-124, Plenum Press; A. P. Blanchard, R. J. Kaiser, L. E. Hood, High-Density Oligonucleotide Arrays, Biosens. & Bioelectronics 11, S. 687, 1996).

Eine alternative Methode ist die ortsaufgelöste Aktivierung von Syntheseplätzen, was z.B. durch selektive Belichtung oder selektive Zugabe von Aktivierungsreagenzien (Entschützungsreagenzien) möglich ist. Die Menge an synthetisierten Molekülen einer Spezies ist bei den bisher bekannten Methoden verhältnismäßig gering, da in einem Mikroarray definitionsgemäß nur jeweils kleine Reaktionsbereiche für jeweils eine Sequenz vorgesehen werden, um möglichst viele Sequenzen im Array und damit für den funktionellen Einsatz abbilden zu können.

Beispiele für die bisher bekannten Verfahren sind die photolithographische lichtgestützte Synthese [McGall, G. et al; J. Amer. Chem. Soc. 119; 5081-5090; 1997], die projektorbasierte lichtgestützte Synthese [PCT/EP99/06317], die fluidische Synthese mittels Trennung der Reaktionsräume, die indirekte projektorbasierte lichtgesteuerte Synthese mittels Photosäuren und geeigneter Reaktionskammern in einem mikrofluidischen Reaktionsträger, die elektronisch induzierte Synthese mittels ortsaufgelöster Entschützung an einzelnen Elektroden auf dem Träger und die fluidische Synthese mittels ortsaufgelöster Deposition der aktivierten Synthese-Monomere.

Die Nutzung von trägergebundenen Bibliotheken ist für die Synthese von synthetischen Genen in PCT/EP00/01356 beschrieben. Ein Nachteil dieses Verfahrens ist, dass die Matrix der Moleküle durch den Schritt des Herauslösens zerstört wird. Ein zweiter wichtiger Punkt ist die Menge an synthetischer DNA, die pro Reaktionsplatz im Träger, also pro Sorte an Oligo, hergestellt werden kann. Durch den kleinen Maßstab, der definitionsgemäß mit der Synthese verbunden ist, ist zudem die Handhabung der herausgelösten Oligonukleotide nicht einfach.

Eine Nutzung von trägergebundenen Nukleinsäuren, die in einer Array-Anordnung hergestellt werden, ist auf der Homepage der Arbeitsgruppe Dr. J. Hoheisel vom Deutschen Krebsforschungszentrum Heidelberg (DKFZ) angegeben. Hier wird z.B. die Nutzung der Nukleinsäuren als PCR-Primer thematisiert. Allerdings ist auch hier die Ablösung der Moleküle direkt vom Träger beschrieben. Eine Nutzung als Matrize ist nicht beschrieben.

Eine weitere Nutzung von trägergebundenen Nukleinsäuren, die in einer Array-Anordnung hergestellt werden, ist in Bulyk et al. [Bulyk, M. et al; Nat. Biotech. 17; 573-577; 1999] beschrieben. In dieser Anwendung werden Mikroarrays mittels der photolithographischen lichtgestützten Synthese der Firma Affymetrix so aufgebaut, dass unterschiedliche 25mere mit freien 5'-Enden auf der festen Phase dargestellt werden. Diese werden dann durch proximal bindende Primer zu Doppelsträngen aufgefüllt. Die Anwendung dieser Doppelstrang-Arrays ist dann die Analyse von Bindungsereignissen mit DNA-bindenden Proteinen. Für Zwecke der Analytik werden zudem enzymatische Verdaue mit Restriktions-Enzymen beschrieben. Eine Nutzung der erzeugten Kopien nach Abtrennung von den als Matrizen dienenden Oligonukleotiden wird nicht beschrieben. Auch ein wiederholtes oder cyclisches Kopieren wird nicht beschrieben. Da das verwendete Syntheseverfahren auf Photolithographie beruht, ist es zudem für den Fachmann ersichtlich, dass für ein neues Array-Design bzw neue Nukleinsäure-Sequenzen erheblicher Aufwand inklusive der Beschaffung entsprechender Masken notwendig ist.

Die Anwendung der photolithographischen Technologie ist für die exakte Belichtung von Mustern während der Synthese sehr gut geeignet. Dadurch wird die routinemäßige parallele Herstellung von high-density-Arrays ermöglicht. Dieser Ansatz ist jedoch einigen Einschränkungen unterworfen, da dieser physikalische anstatt digitale Konstruktionen erfordert. Insbesondere ist die Herstellung von Masken-Sets ein teurer und zeitaufwändiger Prozess. Zusammenfassend unternehmen Bulyk et al. zwar erste Schritte hin zum Ergänzen des Nukleinsäure-Einzelstrangs zum Doppelstrang, arbeiten dann aber nur die Richtung weiterer analytischer Anwendungen dieser Doppelstrang-Arrays (Bindungsassays mit DNA-bindenden Proteinen) und schlagen die anderweitige Nutzung der erzeugten Kopien nach Trennung von der Matrize, deren wiederholte oder cyclische Herstellung oder eine Kombination mit einer Proben-Amplifikation, wie sie als Ausführungsformen des erfindungsgemäßen Verfahrens weiter unten beschrieben sind, weder vor, noch legen sie eine solche Erfindung nahe.

Bekannt ist ferner die festphasengestützte Amplifikation von Ziel-Nukleinsäuren, z.B. dem Pool der mRNA-Moleküle eines biologischen Extraktes [Fa. Linden Bioscience, Veröffentlichung zu "Solid Phase Transkription Chain Reaction" oder "SP-TCR"]. Hierzu wurden zwei verschiedene Primer an eine feste Phasen gekoppelt (eine *in situ* Synthese ist nicht beschrieben und angesichts von zwei Primern für den Fachmann auch nicht naheliegend), die Sequenzen für die viralen RNA Polymerase-Promotoren T3 und T7 enthalten, ferner Elemente für die Hybridisierung von Poly-T RNA in Verbindung mit dem T7 Promoter-Primer. Nach Hybridisierung einer mRNA über den Poly-A Bereich wird der Strang zum Doppelstrang aufgefüllt. Anschließend wird eine spezielle Kassette (TCR-Adapter) an den Doppelstrang ligiert, die ihrerseits eine Erkennungsstelle mit dem T3 Promoter-Primer gemeinsam hat. So entsteht eine Transkriptions-Kettenreaktion. Das Verfahren SP-TCR funktioniert hocheffizient an der festen Phase. Die dem erfindungsgemäßen Verfahren zugrunde liegende Herstellung einer Bibliothek aus Matrizen-Nukleinsäuren als Startpunkt von Kopiervorgängen wird nicht nahegelegt.

In einem ebenfalls festphasengestützen Ansatz zur Amplifikation durch Strand Displacement wurde von Westin et al. [Westin L. et al.; Nat. Biotech 18; 199-204; 2000] gezeigt, dass die parallele Nutzung von mehr als einem Primer an einer gemeinsamen festen Phase mit hoher Effizienz möglich ist. Allerdings werden bei Westin et al. die Primer-Nukleinsäuren getrennt vom Reaktionsträger und nicht *in situ* hergestellt und dann erst dort platziert. Damit entfällt der zentrale Aspekt der hocheffizienten *in situ* Synthese. Außerdem ist kein Hinweis darauf zu finden, dass erst die Kopien der Matrizen-Nukleinsäuren die eigentlichen Reaktionsteilnehmer sind. Vielmehr werden auch die anderen Primer mit der analytspezifischen Sequenz extern hergestellt und dann der Reaktion zugegeben. Eine Kopie der Matrize wird nicht durchgeführt.

### 1.3 Gegenstand der Erfindung

Bereitgestellt werden soll ein Verfahren zur Herstellung einer Mehrzahl von unterschiedlichen synthetischen Nukleinsäuren wahlfreier Sequenz durch Herstellung geeigneter festphasengestützter synthetischer Bibliotheken als Matrizen und eine matrizenabhängige biochemische Kopier-Reaktion.

Damit können Nukleinsäure-Stränge in hoher Ausbeute und gleichzeitig mit sehr vielen unterschiedlichen Sequenzen von einem Träger mit einer Bibliothek darauf abgeschrieben und für weitere Prozessschritte zur Verfügung gestellt werden.

Gegenstand der Erfindung ist demnach ein Verfahren zur enzymbasierten Synthese von Nukleinsäuren durch Kopie einer als Array in einer Matrix synthetisierten Matrizen-Bibliothek, durchgeführt auf einem enzymbasierten Nukleinsäure-Matrix-Synthesizer als Vorrichtung.

Bevorzugte Ausgestaltungen der Erfindung sind in den Ansprüchen 1 bis 31 wiedergegeben.

### 1.4 Grundzüge des Lösungsweges

Die Matrizen für die enzymbasierte Synthese mittels Kopiervorgang bestehen ihrerseits aus kopierfähigen Nukleinsäurepolymeren, die in Form einer Array-Anordnung auf einem gemeinsamen Träger synthetisiert werden. Sie stehen nach ihrer eigentlichen Synthese in kopierfähigem Zustand zur Verfügung und können in einem enzymbasierten Verfahren unter Zugabe entsprechender Reagenzien und Hilfsstoffe, wie Nukleotiden, vervielfältigt werden.

Durch Einsatz bekannter Verfahren zur Herstellung von solchen Arrays aus Nukleinsäurepolymeren, z.B. in Form eines so genannten Mikroarrays, können sehr viele (typischerweise mehr als 10) unterschiedliche Nukleinsäurepolymere von mindestens mehr als 2, typischerweise mehr als 10 Basen Länge, erzeugt werden.

Beispiele für solche Verfahren sind oben beschrieben. Alle diese Verfahren führen letztlich zu einer Bibliothek oder einem Set von Oligonukleotiden oder Polynukleotiden auf einem gemeinsamen Träger. Dies soll unter dem oben genannten Begriff Nukleinsäurepolymere in einer Matrix-Anordnung zusammengefasst werden. Alle diese Verfahren dienen im Wesentlichen der Herstellung so genannter Mikroarrays für die Analyse von Nukleinsäuren mittels Hybridisierung.

Der nächste Schritt im erfindungsgemäßen Verfahren besteht nun darin, die an der festen Phase synthetisierten Moleküle mit Hilfe entsprechender Enzyme zu kopieren. Dazu sind zahlreiche Enzym-Systeme bekannt und kommerziell erhältlich. Beispiele hierfür sind DNA-Polymerasen, thermostabile DNA-Polymerasen, Reverse Transkriptasen und RNA Polymerasen.

Die Reaktionsprodukte zeichnen sich durch große Vielfalt der Sequenz aus, die indirekt über die Matrizen-Moleküle während des vorgeschalteten Synthesevorgangs frei wählbar programmiert werden kann. Ein Mikroarray aus dem Geniom-System kann in einem Kanal als Reaktionsraum 6.000 frei wählbare Oligonukleotide mit einer Sequenz von bis zu 30 Nukleotiden synthetisieren. Nach dem Kopierschritt liegen entsprechend 6.000 frei programmierbare 30mere DNA oder RNA in Lösung vor und können als Reaktanden in einem nächsten Verfahrensschritt oder als Endprodukt zur Verfügung gestellt werden.

Für den Start des Kopierschritts wird es dabei in einigen Ausführungsformen notwendig sein, so genannte Primer-Moleküle zuzugeben, die als Initiationspunkt für Polymerasen dienen. Diese Primer können aus DNA, RNA, einem Hybrid der beiden oder aus modifizierten Basen bestehen. Auch der Einsatz von Nukleinsäure-Analoga, wie PNA- oder LNA-Molekülen als Beispiel, ist in bestimmten Ausführungsformen vorgesehen. Zur Schaffung einer Erkennungsstelle für den Primer kann es zweckmäßig sein, am Ende eines jeden Nukleinsäurepolymeres auf dem Träger eine einheitliche Sequenz hinzuzufügen, entweder als Teil der Synthese oder in einem zusätzlichen Schritt mittels einer enzymatischen Reaktion, wie einer Ligation einer vorgefertigten Nukleinsäure-Kassette. In einer Variante ist das distale Ende der am Träger synthetisierten Sequenz selbstkomplementär und kann so einen hybriden Doppelstrang ausbilden, der von den Polymerasen als Initiationspunkt erkannt wird.

Zweck des Verfahrens ist die Bereitstellung von Nukleinsäuren mit hoher und rationell programmierbarer Diversität der Sequenzen für in einem nächsten Schritt nachfolgende Verfahren. Beispiele für diese Verfahren sind:
- die Herstellung von Primern für Primer Extension Methoden, Strand Displacment Amplifikation, Polymerase Chain Reaction, Site Directed Mutagenesis oder Rolling Circle Amplifikation,
- Genexpressions-Modulation mittels RNAi oder Antisense-Methoden,
- Herstellung oder Vorbereitung von Analyten (sample preparation) für die logisch nachgeordnete Analyse durch Mikroarrays, Sequenzierverfahren, Amplifikationsverfahren (Strand Displacment Amplifikation, Polymerase Chain Reaction oder Rolling Circle Amplifikation) oder Analyse in einer Gelelektrophorese,
- RNA-Bibliotheken für die Translation *in vitro* oder i*n vivo,*
- Klonierung von Sequenzen mittels Vektoren oder Plasmiden,
- Ligation der Nukleinsäuren in Vektoren oder Plasmide,
- Validierung oder Test von Hybridisierungs-Assays und zugehörigen Reagenzien und Kits mittels der erzeugten Nukleinsäurepolymeren in den Bereichen Mikroarrays, Dot blots, Southern- oder Northern-Blots, Bead-Arrays, Serial Analysis of Gene Expression [SAGE],
- Referenz- oder Kalibrier-Verfahren oder Verfahrensschritte innerhalb von Assays aus den Bereichen Mikroarrays, Dot blots, Southern- oder Northern-Blots, Bead-Arrays, Serial Analysis of Gene Expression [SAGE].

Allen diesen Verfahren ist die Nutzung von Nukleinsäuren als hybridisierfähigem Reagenz gemeinsam. Darüber hinaus gibt es auch noch Verfahren, die Nukleinsäurepolymere nicht oder nicht ausschließlich über eine Hybridisierungsreaktion nutzen. Hierzu gehören Aptamere und Ribozyme.

Die Herstellung der Nukleinsäurepolymere bietet an mehreren Stellen des Verfahrens die Möglichkeit, mit bekannten Methoden in die Reaktionsprodukte Modifikationen oder Markierungen einzuführen. Hierzu zählen markierte Nukleotide, die z.B. mit Haptenen oder optischen Markern, wie Fluorophoren und Lumineszenz-Markern, modifiziert sind, markierte Primer oder Nukleinsäure-Analoga mit besonderen Eigenschaften, wie z.B. besondere Schmelztemperatur oder Zugänglichkeit für Enzyme.

Beispiel einer bevorzugten Ausführungsform der Erfindung und Ablauf des Verfahrens:
1. Herstellung eines Mikroarrays aus 6.000 verschiedenen 30meren in einem Gerät geniom® one mit distalem 3'-Ende.
2. Anfügen einer generischen Primer-Sequenz von 15 Basen durch nasschemische statt lichtgesteuerte Synthese an allen 6.000 Oligos abzüglich einer Kontrolle. Die Primer-Sequenz ist so gewählt, dass eine PCR Reaktion möglich ist.
3. Prozessieren des Array bis zu einem hybridisierbereiten Zustand.
4. Zugabe von Primer und Nukleotiden sowie Taq DNA-Polymerase und cyclische Durchführung der PCR-Reaktion entweder direkt im Gerät geniom® one oder in einer Halterung für *in situ* Hybridisierung in einer anderen, kommerziell erhältlichen PCR-Maschine, die allerdings nicht in die exponentielle Phase übergeht.
5. Durchfahren von 10 Zyklen und entsprechend 10faches Abschreiben der festhängenden Matrizen-Oligonukleotiden und finaler Heizschritt zwecks Ablösen des letzten Zweitstrangs.
6. Elution der Kopien in ein Eppendorf-Gefäß. Das Gefäß enthält jetzt 6.000 unterschiedliche programmierte 45mere aus DNA, die für beliebige Applikationen bereit stehen.

### 1.5 Ausführungsformen, Varianten und Anwendungen

### 1.5.1 Reaktionsträger und feste Phasen

Generell können alle solche Reaktionsträger und feste Phasen für das erfindungsgemäße Verfahren genutzt werden, für die eine Synthese einer Matrix aus Nukleinsäurepolymeren als Matrize des Kopiervorgangs etabliert ist.

Dazu gehören als beispielhafte Vertreter die folgenden, dem Fachmann bekannten Reaktionsträger-Formate und feste Phasen:
- Flacher Reaktionsträger, auch "Chip" genannt,
- Poröser Träger,
- Reaktionsträger mit Elektroden,
- Reaktionsträger mit temporär oder permanent immobilisierter fester Phase aus Partikeln oder Beads,
- Mikrofluidischer Reaktionsträger,
- Oberflächen-Modifikation: Gele, Linker, Spacer, Polymere, amorphe Schichten, 3D-Matrizes.

Einige dieser Reaktionsträger können in Kombination verwendet werden, z.B. ein mikrofluidischer Reaktionsträger mit porösen Oberflächen.

### 1.5.2 Bevorzugte Ausführungsformen für die in situ Synthese des Arrays

Der Aufbau der DNA-Sonden findet durch lichtgesteuerte *in situ* Synthese in einem geniom® one Instrument der Fa. febit unter Verwendung moderner Schutzgruppenchemie in einer dreidimensionalen Mikrostruktur als Reaktionsträger statt. In einem zyklischen Syntheseprozess wechseln sich Belichtungen und Kondensationen der Nukleotide so lange ab, bis an jeder Position des Arrays in den Mikrokanälen die gewünschte DNA-Sequenz vollständig aufgebaut wurde. Auf diese Weise können bis zu 48.000 Oligonukleotide mit einer Länge von bis zu 60 Einzelbausteinen hergestellt werden. Die Oligonukleotide binden dabei kovalent an ein Spacermolekül, einen chemischen Abstandhalter auf der Glasoberfläche des Reaktionsträgers. Die Synthese verläuft softwaregesteuert und ermöglicht eine hohe Flexibilität beim Aufbau des Arrays, den der Anwender damit seinen Bedürfnissen entsprechend individuell konfigurieren kann. So können z.B. die Länge der Oligonukleotide, die Zahl der erzeugten Nukleinsäure-Sonden oder interne Kontrollen optimal auf das jeweilige Experiment abgestimmt werden.

Die Kopierreaktion beruht auf einer Primer-Sequenz distal an den Sonden, die zu einem Primer von 15 Basen Länge passt, und durch eine auf allen Oligonukleotiden gleich erfolgten einheitlichen Synthese mittels Standard-DMT Schutzgruppenchemie aufgebaut wurde. Der Reaktionsträger enthält 8 getrennte Reaktionsräume, die individuell genutzt werden können und nicht den gleichen Array enthalten müssen, aber können. In dieser Ausführungsform werden 45mere an der Oberfläche synthetisiert.

Nach abgeschlossener Synthese der Matrizen-Oligonukleotide und finaler Entfernung der Schutzgruppen an den Nukleobasen sind die Arrays bereit für eine Hybridisierung.

Der Reaktionsträger wird entnommen und in eine beheizbare (Peltier-Element) Einheit eingelegt, die einen fluidischen Anschluss, Ventile und eine Pumpe (Kolbenpumpe) enthält. Diese Einheit dient für die Teilautomatisierung von Prozessschritten. Für die Kopierreaktion wird ein Gemisch aus Primer, Biotin-markierten Nukleotiden, Restriktionsenzym zur Einführung von Einzelstrangbrüchen am Primer und DNA-Polymerase hinzugefügt. Nach 4 Stunden Reaktion bei 32 °C wird die Reaktion durch einen einzelnen Heizschritt von 90 °C gestoppt und eine Denaturierung aller vorhandenen Doppelstränge herbeigeführt. Da von 45mer Oligonukleotiden abgeschrieben wurde, liegt nun ein Reaktionsgemisch vor, das an Nukleinsäuren in Lösung einmal die restlichen Primer (15mere) und zum zweiten ein Set von 45meren enthält. Die 45mere enthalten alle am 5'-Ende die komplementäre Sequenz des Primers, am 3'-Ende jedoch 30 völlig frei wählbare Basen.

Diese Basen-Sequenz wird so gewählt, dass jeweils zwei 45mere ein Primer-Paar für eine nun nachfolgende Reaktion bilden. Diese Primer liegen beide außerhalb eines zu analysierenden SNP auf einer Zielsequenz und haben einen Abstand von 1-30 Basen.

### 1.5.3 Initiation des Kopiervorgangs an den Matrizen-Nukleinsäuren

Die Initiation an den Matrizen-Nukleinsäuren kann prinzipiell mit allen Methoden erfolgen, die dem Fachmann für die Initiation eines enzymatischen Kopiervorgangs von Nukleinsäuren bekannt sind, also z.B. aus den Anwendungen *Polymerase Chain Reaction, Strand Displacement* und *Strand Displacement Amplification, in vitro* Replikation, Transkription, Reverse Transkription oder virale Transkription (Vertreter hiervon sind T7 und SP6).

In einer Ausführungsform wird ein T7 oder ein SP6 Promoter in einen Teil oder alle Nukleinsäurepolymere am Reaktionsträger eingefügt.

In einer anderen Ausführungsform dient der Array aus Nukleinsäuren der Initiation einer isothermalen Kopierreaktion. Ein Vertreter dieser Verfahren ist die Strand Displacement Reaktion. Hiervon sind in der Literatur zahlreiche Varianten bekannt. Dazu wird z.B. ein Primer gewählt, der an die Matrizen-Polymeren an deren distalem Ende bindet und dann dort in 3'-Richtung verlängert werden kann. Alle oder ein bestimmter Teil der Nukleinsäurepolymere auf dem Träger enthalten distal diese Primer-Sequenz. Als nächstes wird ein Enzym zugegeben, für das der Primer eine Erkennungsstelle enthält, so dass ein Einzelstrangbruch induziert wird. Die übliche Vorgehensweise sieht dazu die Verwendung einer Restriktionsnuklease vor, z.B. N.NBstNB I (erhältlich z.B. von Fa. von New England Biolabs), das von Natur aus nur Einzelstrangbrüche (so genannte Nicks) einführt, da es keine Dimere bilden kann.

### 1.5.4 Variante mit Rolling Circle

Gemäß einer weiteren Ausführungsform der vorliegenden Erfindung werden doppelsträngige, ringförmige Nukleinsäurefragmente bereitgestellt, wobei ein Strang an der Oberfläche des Trägers verankert wird und der andere Strang ein selbstprimendes 3'-Ende umfasst, so dass eine Elongation des 3'-Endes erfolgen kann. Die enzymatische Synthese umfasst bei dieser Variante des erfindungsgemäßen Verfahrens eine Replikation analog des für die Replikation von Bakteriophagen bekannten Rolling Circle-Mechanismus, wobei ein Strang der ringförmigen Nukleinsäurefragmente an der Oberfläche des Trägers verankert ist und mehrfach kopiert werden kann. Wenn zunächst ein doppelsträngiges geschlossenes Nukleinsäure-Fragment vorliegt, kann der zweite Strang zunächst durch einen Einzelstrangbruch geöffnet werden, wobei ein 3'-Ende gebildet wird, von welchem ausgehend die Elongation stattfindet. Die Abspaltung des elongierten Strangs kann z.B. enzymatisch erfolgen. Durch Zugabe von Nukleotidbausteinen und eines geeigneten Enzyms erfolgt dann eine Synthese der jeweils zu den Basensequenzen der an der Oberfläche des Trägers verankerten Nukleinsäurestränge komplementären Teilsequenzen.

### 1.5.5 Label, Bindungsstellen und Marker

Die Produkte des Kopiervorgangs können auf verschiedenen Wegen Label, Bindungsstellen oder Marker erhalten, die für eine weitere Prozessierung oder den Einsatz in weiteren Assays oder Verfahren erwünscht sind.

Dazu gehören Marker und Label, die eine direkte Detektion der Kopien erlauben und dem Fachmann aus anderen Verfahren zur Kopie von Nukleinsäuren bekannt sind. Beispiel hierfür sind Fluorophore. Des Weiteren können Bindestellen für indirekte Nachweisverfahren oder Reinigungsverfahren vorgesehen werden. Dazu zählen als Beispiele Haptene, wie Biotin oder Digoxigenin.

Die Label, Bindungsstellen oder Marker können in einer Variante durch modifizierte Nukleotide eingeführt werden. Ein weiterer Weg eröffnet sich bei Verwendung von Primern für die Initiation des Kopiervorgangs. Die Primer können bereits mit Label, Bindungsstellen oder Marker in die Reaktion eingebracht werden.

Nachträglich können Label, Bindungsstellen oder Marker eingebracht werden, indem die Reaktionsprodukte einer nachfolgenden Markierungsreaktion mit generischen, mit den Nukleinsäuren reagierenden Agenzien behandelt werden. Ein Beispiel hierfür sind Cis-Platin-Reagenzien. Alternativ hierzu lassen sich Label, Bindungsstellen oder Marker auch durch eine weitere enzymatische Reaktion einführen, wie z.B. durch eine terminale Transferase katalysiert.

### 1.5.6 Integration von Probenvorbereitung und Amplifikation mit einem analytischen Mikroarray

In den hier beschriebenen Ausführungsformen der Erfindung ist das Ziel die Integration der Sample-Amplifikation und der Sample-Analytik auf ein und demselben Festphasenträger (Biochip).

Bislang erfordert die Analytik von DNA- und RNA-Proben in einem ersten Schritt - sowohl bei Genexpressionprofil-Erstellung als auch bei Genotypisierung (SNP-Typisierung, Resequenzierung etc.) - eine Amplifikation der zu untersuchenden Probe. Erst in einem zweiten Schritt kann die zu untersuchende Probe auf einem Biochip über eine Vielzahl von DNA- oder RNA-Rezeptoren hochparallel untersucht werden. Dieses Vorgehen ist zeitintensiv und kostspielig. Mit der hier beschriebenen Variante des erfindungsgemäßen Verfahrens kann dies gelöst werden.

Ein Beispiel für den Stand der Technik ist in EP 1 056 884 (Verfahren zur nicht-spezifischen Amplifikation von Nukleinsäuren (Van Gemen, PamGene B.V.); u.a. oligo-dT-Sequenz am 3'-Ende blockiert) beschrieben. Ein weiteres findet sich in der Veröffentlichung zur "Solid Phase Transkription Chain Reaction" oder "SP-TCR" der Fa. Linden Bioscience.

### 1.5.6.1 Variante 1: Untersuchung von RNA Analyten unter Verwendung von RNA Polymerase und RnaseH

Beispielhafter Ablauf einer integrierten Amplifikation einer Ausführungsform der integrierten Probenvorbereitung und Analyse an einem Träger mit Mikroarray aus Nukleinsäure-Sonden :
- An der Festphase werden spezifische Sonden A_{1....n} für die Amplifikation des zu untersuchenden Targets und spezifische Sonden B_{1.....x} für die Analyse des zu untersuchenden Targets aufgebaut.
- Die den Promotor beinhaltenden Sonden A_{1.....n} sind an ihrem 3'-Ende nicht verlängerbar (entweder Blockierung oder Festphase am 3'-Ende).
- Die Sonden A_{1....n} beinhalten eine Promotor-Sequenz (z.B. T3, T7, SP6), die es erlaubt, bei einer spezifischen Hybridisierung mit den zu untersuchenden Targetsequenzen, diese durch eine RNA-Polymerase zu amplifizieren.
- RNase-H schneidet den RNA-Teil des DNA-RNA-Duplex am 3'-Ende ; es wird darauf folgend dort ein doppelsträngiger (dsDNA) Promotor(T7, Sp6, T3) aufgebaut.
- Ausgehend vom dsDNA-Promotor werden die zu den Targetsequenzen komplementären RNA-Sequenzen C_{1.....y} in großer Zahl hergestellt (antisense-RNA).
- Die amplifizierten RNA-Targetsequenzen C_{1.....y} können nun nach Hybridisierung an die spezifischen Sonden B_{1.....x}analysiert werden.

Die Analyse der Wechselwirkung der Targetsequenzen C_{1.....y} mit den spezifischen Sonden B_{1......x} kann über ein Hybridisierung-vermitteltes Verfahren oder auch über ein Enzym-vermitteltes Verfahren erfolgen.

Zur Amplifikation wird eine RNA-Polymerase mit RNaseH-Aktivität eingesetzt (z.B. AMV-RT, MLV-RT); Mix aus rNTPs, dNTPs.

Beispielhaftes Vorgehen:
- Die Spannweite der Umsetzung ist weit, das Design hat ein große Flexibilität hinsichtlich:
- Orientierung Sonden A_{1....n}: 5'-3' oder 3'-5'
- Orientierung Sonden B_{1.....x}: 3'-5' oder 5'-3'
- Art des Targets: RNA (bei DNA müssten RNA-Sonden aufgebaut werden)
- Analytisches Wirkprinzip: Enzym (Einbau Signalgeber währendEnzymreaktion) oder Hybridisierung (Einbau Signalgeber während Amplifikationsschritt)

### 1.5.6.2 Variante 2: Untersuchung von RNA Analyten unter Verwendung von RNA Polymerase

Hierfür wird in Analogie zu SP-TCR (siehe oben) eine Transcription Chain Reaction gestartet. Dazu werden in den Matrizen-Nukleinsäuren in geeigneter Orientierung und unter Berücksichtigung von sense/antisense Erfordernissen sequenzspezifische Primer-Abschnitte mit RNA-Polymerase-Promotoren kombiniert. Gut etablierte Vertreter der viralen RNA-Polymerase- Promotoren sind T7, T3 und SP6. Dabei liegt der RNA-Promoter jeweils proximal zur festen Phase und der sequezspezifische Abschnitt, der der selektiven Erkennung seines komplementären Abschnittes in den Ziel-Nukleinsäuren dient, distal vom Träger. So kann als Beispiel in einem Experiment zur Analyse der mRNA-Population einer untersuchten Probe in einem Array von 6.000 verschiedenen DNA-Oligos (siehe geniom® one von Fa. febit) für bis zu 6.000 verschiedene Sequenz-Abschnitte ein geeigneter Primer-Oligo bereitgestellt werden. Soll pro Gen eine Amplifikation initiiert werden, lassen sich auf diese Weise Amplicons für 6.000 Gene parallel in einer Reaktion herstellen. In einer weiteren Ausführungsform werden hingegen 2 Primer für jedes Gen verwandt, die aber je einer von 2 zu verwendenden Promotoren (z.B. T7 und T3) enthalten. So kann eine Gen-spezifische TCR induziert werden, die in einer einzigen Reaktion exponentiell 3.000 Amplicons für 3.000 Gene herstellt. Dieses Reaktionsprodukt kann nun in beliebigen weiteren Verfahren analysiert werden. Eine bevorzugte Analyse ist eine Hybridisierungsreaktion auf einem Mikroarray.

In einer anderen Ausführungsform werden lineare oder exponentielle Transkriptions-Amplifikation mit entsprechenden analytischen Sonden (wie oben beschrieben) kombiniert.

### 1.5.6.3 Variante 3: Erzeugung von sequenzspezifischen Primern in Lösung für die Verlängerung in Abhängigkeit von Zielmolekülen (Targetanalyt) in der Probe

In dieser Ausführungsform werden die Kopien der Matrizen-Nukleinsäuren ihrerseits zur Reaktion mit den Ziel-Nukleinsäuren verwendet. Dabei werden die Sequenzen so gewählt, dass die später in einer Hybridisierreaktion zu analysierende Sequenz erst bei erfolgreicher Verlängerung der einzelnen kopierten in Lösung befindlichen Nukleinsäurepolymere entsteht. Diese Abschnitte können dann ihrerseits mittels eines Arrays detektiert werden. In der bevorzugten Ausführungsform geschieht dies wie oben beschrieben auf mittels analytischer Sonden im gleichen Mikroarray oder auf einem fluidisch verbundenen Array.

In einer Variante zur Erzeugung des Signals kann es vorgesehen sein, dass die Primer zur Initiation des Kopiervorgangs bereits eine Modifikation tragen, die die Erzeugung des Signals unterstützt. Ein Beispiel für eine solche Modifikation ist ein Primer, der in seinem 5'-Abschnitt in einem Bereich, der nicht für die Hybridisierung mit der Matrize notwendig ist, eine branched-DNA-Struktur trägt [Collins M.L. et al.; Nucleic Acids Res. 25(15); 2979-2984; 1997).

Eine andere Variante sieht vor, dass für jede Zielsequenz, also z.B. ein einzelnes Gen oder Exon, zwei Primer mit gegenläufiger Spezifität bereitgestellt werden, so dass in einer PCR oder isothermalen Amplifikation eine effiziente exponentielle Vervielfältigung erfolgt.

Bei gleichzeitiger Reaktion von Kopiervorgang, Amplifikation und Hybridisierung an die analytischen Sonden kann in einem sehr kompakten und simplifizierten Format die komplette Analyse eines Gemisches an Ziel-Nukleinsäuren durchgeführt werden. Solch eine komplette Analyse kann z.B. die Detektion aller in einer Gesamt-RNA Probe aus einem biologischen Untersuchungsobjekt wie einer Zellkulturpopulation oder einer Tumorbiopsie enthaltenen exprimierten Gene aufklären - ohne vorherige Proben-Amplifikation und mit sehr einfacher Probenvorbereitung mit Standard-Kits, wie sie von diversen Herstellern angeboten werden.

Eine dazu gehörige Vorrichtung besteht aus
- einem Gerät für die *in situ* Synthese der Arrays von Matrizen-Polymeren und analytischen Sonden,
- einer Detektionseinheit für die Erfassung eines optischen oder elektrischen Signals,
- einer speicherprogrammierbaren Einheit für die Steuerung der Synthese,
- einer speicherprogrammierbaren Einheit für die Steuerung der Detektion und die Speicherung und Verwaltung der Messdaten,
- optional: Elemente für die Abarbeitung fluidischer Schritte, wie der Probenzugabe oder/und Probenableitung,
- optional: Elemente für die Automatisierung der Probenaufbereitung aus möglichst unbehandeltem biologischen Untersuchungsgut, also z.B Zelllyse und Reinigung der Nukleinsäuren,
- optional: Einrichtung für die automatisierte Übertragung der aufbereiteten Probe in oder auf den Reaktionsträger.

### 1.5.6.4 Signalerzeugung bei Integration von Matrizen-gesteuerter Amplifikation mit einem analytischen Mikroarray

Beispiele für Signale, die bei der Analyse der Reaktionsergebnisse und der Hybridisierung an die dafür vorgesehenen Nukleinsäurepolymere (analytische Sonden) auf dem Reaktionsträger bzw Array genutzt werden können, sind unter anderem die in der Fachwelt gut bekannten folgenden Signale:
- Optische Signale
   o Fluoreszenz (organische und anorganische Fluorophore),
   o Lichtstreuung (z.B. Goldpartikel in nm-Dimensionen),
   o Chemilumineszenz,
   o Biolumineszenz;
- Elektrische Signale
   o Stromfluss,
   o Redoxreaktionen.

Die Signale können dabei durch Labels, Bindungsstellen oder Marker, ähnlich den oben beschriebenen, in die Reaktionsprodukte eingeführt werden. Dabei können einerseits die Kopien der Matrizen-Nukleinsäuren entsprechend behandelt werden. In einer alternativen Ausführungsform werden die Labels, Bindungsstellen oder Marker während einer weiteren Reaktion in die Ziel-Analyten eingebracht.

Ein Beispiel hierfür ist eine Verlängerung von Primern, die selber Reaktionsprodukte des Kopiervorgangs sind, in Abhängigkeit von Ziel-Nukleinsäuren (Analyten) in der Probe, an die sie für diese Reaktion hybridisieren können, so dass nur bei spezifischer Hybridisierung eine Verlängerung zustande kommt. Während dieser Verlängerung werden dann die Labels, Bindungsstellen oder Marker in diese verlängerten Polymere eingeführt, so dass anschließend deren Bindeverhalten auf dem Array in Zusammenhang mit den analytischen Sonden beobachtet und ausgewertet werden kann.

In einer weiteren Ausführungsform werden die verlängerten Polymere mit analytischen Nukleinsäure-Sonden in Kontakt gebracht, die wiederum für eine Verlängerung in Form einer Primer-Extension nutzbar sind. Die Anordnung eines Primer-Extension Experimentes ist aus der Fachliteratur bekannt. Das Signal der Primer-Extension an diese Analyse-Sonden wird dann zur Bestimmung des Analyse-Ergebnisses ausgewertet. Eine solche Analyse kann z.B. die Bestimmung von Einzelnukleotid-Polymorphismen (SNP's) in genomischer DNA sein. Dazu werden erst verlängerbare Primer an Matrizen-Nukleinsäuren abkopiert. Die Sequenz ist so gewählt, dass im 3'-Bereich nach der Primer-Sequenz auf der Ziel-Nukleinsäure die zu untersuchenden SNP's lokalisiert sind. Im nächsten Schritt werden diese Primer über die Sequenz der zu detektierenden SNP's hinweg verlängert. Anschließend werden die Reaktionsprodukte dieser Verlängerung durch Primer-Extension oder direkt durch Hybridisierung untersucht und die Ergebnisse für die Bestimmung der in der Analyse abgefragten SNP's registriert. In der speicherprogrammierbaren Vorrichtung werden für den Nutzer der erfindungsgemäßen Vorrichtung die Daten so aufbereitet, dass er z.B. direkt einen Report mit den Basen-Positionen und den gefundenen Basen erhält.

Der große Vorteil der Erfindung liegt dabei darin, dass für solche Genotypisierungs- oder SNP-Analyse-Assays nur noch eine universelle, generische Probenvorbereitung notwendig ist. Primer und Reagenzien, die für einzelne Genotypen oder SNP's spezifisch sind, werden nicht benötigt, da alle Sequenz-Spezifität aus der *in situ* Synthese des zugrunde liegenden Matrizen-Arrays und dem Analyse-Array stammt. In der Ausführungsform mit der Kombination dieser beiden in einem Reaktionsträger wird die Genotypisierung und SNP-Analyse damit maximal vereinfacht.

### 1.5.7 Validierung von Arrays aus Nukleinsäuren

Die eingangs beschriebene Anwendung von Nukleinsäuren und in bestimmten Ausführungsformen von synthetischen Oligonukleotiden in Arrays, bei denen die Moleküle als Rezeptoren oder Fängermoleküle in Reihen und Spalten angeordnet sind, ist allgemein mit der sehr schwierigen empirischen Validierung der hergestellten Arrays unter Zuhilfenahme entsprechender Proben-Moleküle konfrontiert. Dieses Problem ist dem Fachmann gut bekannt und wird mit der Anordnung von mehreren tausend Fängermolekülen in einem Array zu einem zunehmend schwierig lösbaren Problem. Für die Entwicklung von so genannten hochdichten Arrays mit über 100.000 einzelnen Reaktionsräumen ist kein geeignetes rationelles Validierungsverfahren bekannt. Als Notlösung werden schlecht beschreibbare biologisch gewonnene Proben verwendet.

### 1.5.8 Synthetische Gene

In einer Ausführungsform werden qualitativ hochwertige und in der Sequenz frei programmierbare Nukleinsäuren in Form von Oligonukleotiden mit 10-200 Basen Länge kostengünstig und effizient in einer Vielfalt von 10 oder mehr unterschiedlichen Sequenzen bereitgestellt, um synthetische kodierende doppelsträngige DNA (synthetische Gene) herzustellen.

Der Aufbau von doppelsträngiger DNA aus Oligonukleotiden ist seit den 60er Jahren des letzten Jahrhunderts bekannt [Arbeiten von Khorana und anderen; siehe "Shabarova: Advanced Organic Chemistry of Nucleic Acids", VCH Weinheim]. Er geschieht in der Mehrzahl der Fälle mit einem von zwei Verfahren [siehe Holowachuk et. al., PCR Methods and Applications, Cold Spring Harbor Laboratory Press]:

Einmal erfolgt die Synthese des gesamten Doppelstrangs durch Synthese von einzelsträngigen Nukleinsäuren (geeigneter Sequenz), Aneinanderlagerung durch Hybridisieren komplementärer Bereiche dieser Einzelstränge und Verbinden des molekularen Rückgrats durch Enzyme, meist Ligase.

Demgegenüber gibt es auch die Möglichkeit einer Synthese von an den Rändern überlappenden Bereichen als einzelsträngige Nukleinsäuren, Aneinanderlagerung durch Hybridisieren, Auffüllen der einzelsträngigen Bereiche durch Enzyme (Polymerasen) und dann Verbinden des Rückgrats durch Enzyme, meist Ligase.

Ein bevorzugter Ablauf einer Gensynthese gemäß der Erfindung ist wie folgt: Allgemein wird im Rahmen eines modularen Systems eine Synthese vieler einzelner Nukleinsäurestränge durch Anwendung des erfindungsgemäßen Verfahrens zur hochparallelen DNA-Synthese auf Matrizenbasis durchgeführt. Es entstehen als Reaktionsprodukte Sets von Nukleinsäuren, die als Bausteine in einem nachgeschalteten Prozess dienen. Damit wird eine Sequenz-Matrix erzeugt, die über 100.000 unterschiedliche Sequenzen enthalten kann. Die Nukleinsäuren liegen in einzelsträngiger Form vor und können aus dem Träger eluiert werden oder direkt im Reaktionsträger zur Reaktion gebracht werden. Durch wiederholtes Kopieren in einem oder mehreren Arbeitsgängen kann die Matrize mehrfach abgeschrieben werden, ohne diese zu zerstören, und gleichzeitig wird eine Vermehrung der jeweiligen in der Matrix kodierten Sequenzen erreicht. Wie an anderer Stelle ausführlicher beschrieben, kann durch Kopieren von distal nach proximal dabei auch der Anteil an verkürzten Nukleinsäurepolymeren an der festen Phase ausgeblendet werden, wenn distal die Kopier-Initiationsstelle liegt. Eine Beispiel hierfür ist eine distal angefügte Promoter-Sequenz.

Der Träger mit der Matrix an festphasengebundenen Molekülen kann für spätere erneute Verwendung aufbewahrt werden. Somit wird in einer effizienten Weise die Vielfalt an Sequenzen, die in einem Reaktionsträger durch eine *in situ* Synthese erzeugt wurde, für weitere Prozessschritte zur Verfügung gestellt. Gleichzeitig kann durch das Design der Kopierreaktion eine hohe Qualität der abgeschriebenen Sequenzen erzielt werden.

Danach werden geeignete Kombinationen der abgelösten DNA-Stränge gebildet. Die Zusammenlagerung der einzelsträngigen Bausteine zu doppelsträngigen Bausteinen erfolgt innerhalb eines Reaktionsraums, der in einem einfachen Ansatz ein übliches Reaktionsgefäß, z.B. ein Plastikröhrchen, sein kann. In einer anderen bevorzugten Ausführungsform ist der Reaktionsraum Teil des Reaktionsträgers, der in einer Variante ein mikrofluidischer Reaktionsträger sein kann, in dem die notwendigen Reaktionen ablaufen. Ein weiterer Vorteil eines integrierten mikrofluidischen Reaktionsträgers ist die Möglichkeit der Integration weiterer Prozessschritte, wie z.B. eine Qualitätskontrolle durch optische Analytik. In einer Ausführungsform hat bereits die Synthese der Matrix in einem mikrofluidischen Träger stattgefunden, der dann gleichzeitig als Reaktionsraum für die nachgeschaltete Zusammenlagerung nutzbar ist.

Die Sequenz der einzelnen Bausteine wird dabei so gewählt, dass beim Inkontaktbringen der einzelnen Bausteine zueinander komplementäre Bereiche an den beiden zusammengebrachten Enden zur Verfügung stehen, um durch Hybridisierung dieser Bereiche eine spezifische Aneinanderlagerung von DNA-Strängen zu ermöglichen. Damit entstehen längere DNA-Hybride. Das Phosphodiester-Rückgrat des DNA-Moleküls wird durch Ligasen geschlossen. Sollten die Sequenzen so gewählt werden, dass in diesen Hybriden einzelsträngige Lücken bestehen, so werden diese Lücken in bekannter Vorgehensweise enzymatisch mittels Polymerasen aufgefüllt. (z.B. Klenow-Fragment oder Sequenase). So entstehen längere doppelsträngige DNA-Moleküle. Sollte es für die weitere Verwendung notwendig sein, diese verlängerten DNA-Stränge als Einzelstränge vorzulegen, so kann dies mit den dem Fachmann bekannten Verfahren zum Aufschmelzen von DNA-Doppelsträngen geschehen, wie z.B. Temperatur oder Alkali.

In einer weiteren Ausführungsform können die hergestellten Nukleinsäuren bzw. Nukleinsäure-Doppelstränge während der stufenweisen Kombination und Zusammenlagerung oder im Anschluss daran stabilisiert, kondensiert oder topologisch manipuliert werden. Die Stabilisierung, Kondensation oder topologische Manipulation kann durch funktionelle Moleküle wie Histone oder Topoisomerasen erfolgen.

Durch die Zusammenführung von Clustern an derart synthetisierten DNA-Strängen innerhalb von Reaktionsräumen können wiederum längere Teilsequenzen des finalen DNA-Moleküls erzeugt werden. Dies kann stufenweise geschehen, und die Teilsequenzen werden dabei zu immer längeren DNA-Molekülen zusammengesetzt. Auf diese Weise lassen sich sehr lange DNA-Sequenzen als vollsynthetisches Molekül mit einer Länge von über 100.000 Basenpaaren erzeugen. Dies entspricht bereits dem Größenbereich eines *Bacterial Artificial Chromosome* BAC. Für den Aufbau einer Sequenz von 100.000 Basenpaaren aus überlappenden Bausteinen von 20 Nukleotiden Länge werden 10.000 individuelle Bausteine benötigt. Dies kann mit den meisten der eingangs beschriebenen hochparallelen Synthese-Verfahren geleistet werden. Besonders bevorzugt sind für das erfindungsgemäße Verfahren dabei diejenigen Technologien, die den Array aus Nukleinsäurepolymeren in einer weitgehend frei programmierbaren Weise erzeugen und nicht auf das Einrichten von technischen Komponenten, wie z.B. photolithographischer Masken, angewiesen sind. Demnach sind besonders bevorzugte Ausführungsformen auf die projektorbasierte lichtgestützte Synthese, die indirekte projektorbasierte lichtgesteuerte Synthese mittels Photosäuren und Reaktionskammern in einem mikrofluidischen Reaktionsträger, die elektronisch induzierte Synthese mittels ortsaufgelöster Deprotektion an einzelnen Elektroden auf dem Träger und die fluidische Synthese mittels ortsaufgelöster Deposition der aktivierten Synthese-Monomere aufgebaut.

Für die rationelle Bearbeitung genetischer Moleküle und die systematische Erfassung aller möglichen Varianten müssen die Bausteine in ihrer individuellen Sequenz flexibel und ökonomisch hergestellt werden. Dies leistet das Verfahren durch den Einsatz einer programmierbaren Lichtquellenmatrix für die lichtabhängige ortsaufgelöste *in situ* Synthese der DNA-Stränge, die als Bausteine Verwendung finden. Diese flexible Synthese erlaubt die freie Programmierung der individuellen Sequenzen der Bausteine und damit auch die Erzeugung von beliebigen Varianten der Teilsequenzen oder der finalen Sequenz, ohne dass damit wesentliche Veränderungen von Komponenten des Systems (Hardware) verbunden wären. Nur durch diese programmierte Synthese der Bausteine und damit der finalen Syntheseprodukte kann die Vielfalt genetischer Elemente systematisch bearbeitet werden. Gleichzeitig erlaubt die Verwendung der computergesteuert programmierbaren Synthese die Automatisierung des gesamten Prozesses inklusive der Kommunikation mit entsprechenden Datenbanken.

Die Auswahl der Sequenz der einzelnen Bausteine kann bei Vorgabe der Zielsequenz rationell unter Berücksichtigung von biochemischen und funktionellen Parametern erfolgen. Dabei sucht ein Algorithmus nach Eingabe der Zielsequenz (z.B. aus einer Datenbank) die geeigneten Überlappungsbereiche heraus. Je nach der Aufgabenstellung können unterschiedlich viele Teilsequenzen erstellt werden, und zwar innerhalb eines zu belichtenden Reaktionsträgers oder auf mehrere Reaktionsträger verteilt. Die Anlagerungsbedingungen für die Bildung der Hybride, wie z.B. Temperatur, Salzkonzentration etc., werden durch einen entsprechenden Algorithmus auf die zur Verfügung stehenden Überlappungsbereiche abgestimmt. So wird eine maximale Spezifität der Aneinanderlagerung gewährleistet. Die Daten für die Zielsequenz können in einer vollautomatischen Version auch direkt aus öffentlichen oder privaten Datenbanken entnommen und in entsprechende Zielsequenzen umgewandelt werden. Die entstehenden Produkte können wiederum optional in entsprechend automatisierte Abläufe eingespeist werden, z.B. in die Klonierung in geeigneten Zielzellen.

Der stufenweise Aufbau durch Synthese der einzelnen DNA-Stränge in Reaktionsbereichen innerhalb umgrenzter Reaktionsräume erlaubt auch den Aufbau schwieriger Sequenzen, z.B. solche mit internen Wiederholungen von Sequenzabschnitten, wie sie z.B. bei Retroviren und entsprechenden retroviralen Vektoren vorkommen. Durch die Ablösung der Bausteine innerhalb der fluidischen Reaktionsräume kann eine Synthese beliebiger Sequenz erfolgen, ohne dass Probleme durch die Zuordnung der überlappenden Bereiche auf den einzelnen Bausteinen entstehen.

Die hohen Qualitätsanforderungen, die beim Aufbau sehr langer DNA-Moleküle notwendig sind, werden u.a. durch die Verwendung einer Echtzeit-Qualitätskontrolle erfüllt. Dabei wird die ortsaufgelöste Synthese der Bausteine überwacht, ebenso die Ablösung und die Zusammenlagerung bis hin zur Erstellung der finalen Sequenz. Dann laufen alle Prozesse in einem lichtdurchlässigen Reaktionsträger ab. Des Weiteren wird die Möglichkeit geschaffen, im Durchlicht Reaktionen und fluidische Vorgänge durch z.B. eine CCD-Detektion zu verfolgen.

Der miniaturisierte Reaktionsträger wird so ausgeführt, dass ein Ablösevorgang in den einzelnen Reaktionsräumen möglich ist, und somit die auf den innerhalb dieser Reaktionsräume liegenden Reaktionsbereichen synthetisierten DNA-Stränge in Clustern abgelöst werden. Bei geeigneter Ausführung des Reaktionsträgers ist die Zusammenlagerung der Bausteine in einem stufenweisen Prozess in Reaktionsräumen möglich, außerdem die Entnahme von Bausteinen, Teilsequenzen oder des finalen Produktes, oder auch die Sortierung bzw. Auftrennung der Moleküle.

Die Zielsequenz kann nach ihrer Fertigstellung als integriertes genetisches Element durch Transfer in Zellen eingebracht und dadurch kloniert und im Zuge funktioneller Studien untersucht werden. Eine weitere Möglichkeit ist es, das Syntheseprodukt zuerst weiter aufzureinigen oder zu analysieren, wobei diese Analyse z.B. eine Sequenzierung sein kann. Der Prozess der Sequenzierung kann auch durch direkte Kopplung mit einem entsprechenden Gerät beginnen, z.B. mit einer nach dem in der Patentanmeldung DE 199 24 327 arbeitenden Vorrichtung zur integrierten Synthese und Analyse von Polymeren. Es ist ebenfalls denkbar, die erzeugten Zielsequenzen nach der Klonierung zu isolieren und zu analysieren.

In einer Ausführungsform können die hergestellten Nukleinsäuren bzw. Nukleinsäure-Doppelstränge als propagierbarer Klonierungsvektor verwendet werden, wobei der propagierbare Klonierungsvektor in geeigneten Zielzellen der Transkription, der Expression der transkribierten Sequenz und ggf. der Gewinnung von exprimierten Genprodukten dienen kann.

Das erfindungsgemäße Verfahren stellt über die damit erzeugten integrierten genetischen Elemente ein Werkzeug zur Verfügung, das für die Weiterentwicklung der molekularen Biologie die biologische Vielfalt in einem systematischen Prozess erfasst. Die Erzeugung von DNA-Molekülen mit gewünschter genetischer Information ist damit nicht mehr der Engpass molekularbiologischer Arbeiten, da von kleinen Plasmiden über komplexe Vektoren bis zu Mini-Chromosomen alle Moleküle synthetisch erzeugt werden können und für weitere Arbeiten zur Verfügung stehen.

Das Herstellungsverfahren erlaubt die parallele Erzeugung von zahlreichen Nukleinsäuremolekülen und damit einen systematischen Ansatz für Fragestellungen betreffend Regulationselemente, DNA-Bindestellen für Regulatoren, Signalkaskaden, Rezeptoren, Wirkung und Interaktionen von Wachstumsfaktoren etc.

Durch die Integration von genetischen Elementen in eine vollsynthetische Gesamt-Nukleinsäure können die bekannten genetischen Werkzeuge, wie Plasmide und Vektoren, weiter genutzt und es kann so auf die entsprechenden Erfahrungen aufgebaut werden. Andererseits werden sich diese Erfahrungen durch die angestrebte Optimierung der vorhandenen Vektoren etc. rasch verändern. Die Mechanismen, die z.B. ein Plasmid für die Propagation in einem bestimmten Zelltyp geeignet machen, lassen sich auf der Grundlage des erfindungsgemäßen Verfahrens erstmals rationell untersuchen.

Durch diese rationelle Untersuchung großer Varianten-Zahlen lässt sich der gesamte Kombinationsraum genetischer Elemente erschließen. Damit wird neben die zur Zeit in rascher Entwicklung befindliche hochparallele Analytik (u.a. auf DNA-Arrays oder DNA-Chips) als zweites wichtiges Element die programmierte Synthese integrierter genetischer Elemente geschaffen. Nur beide Elemente zusammen können das Fundament einer rationellen molekularen Biologie bilden.

Bei der programmierten Synthese von entsprechenden DNA-Molekülen ist nicht nur die beliebige Zusammensetzung der kodierenden Sequenzen und funktionellen Elemente möglich, sondern auch die Anpassung der Zwischenbereiche. Dies sollte rasch zu Minimal-Vektoren und Minimal-Genomen führen, womit wiederum Vorteile durch die geringere Größe entstehen. Übertragungsvehikel, wie z.B. virale Vektoren, können dadurch effizienter gemacht werden, z.B. bei Verwendung von retroviralen oder adenoviralen Vektoren.

Über die Kombination bekannter genetischer Sequenzen hinaus ist die Entwicklung neuer genetischer Elemente möglich, die auf der Funktion vorhandener aufbauen kann. Gerade für solche Entwicklungsarbeiten ist die Flexibilität des Systems von enormem Wert.

Die synthetischen DNA-Moleküle sind dabei auf jeder Stufe der Entwicklung des hier beschriebenen Verfahrens voll kompatibel mit der vorhandenen Rekombinationstechnologie. Auch für "traditionelle" molekularbiologische Anwendungen können integrierte genetische Elemente bereitgestellt werden, z.B. durch entsprechende Vektoren. Der Einbau entsprechender Schnittstellen auch für bisher wenig verwendete Enzyme ist bei integrierten genetischen Elementen kein limitierender Faktor.

Ermöglicht wird mit diesem Verfahren die Integration aller gewünschten funktionellen Elemente als "genetische Module", wie z.B. Gene, Teile von Genen, Regulationselemente, virale Verpackungssignale etc., in das synthetisierte Nukleinsäuremolekül als Träger genetischer Information. Durch diese Integration ergeben sich u.a. folgende Vorteile:

Es können damit hochgradig funktionsintegrierte DNA-Moleküle entwickelt werden, wobei unnötige DNA-Bereiche wegfallen (Minimal-Gene, Minimal-Genome).

Die freie Kombination der genetischen Elemente sowie die Veränderungen der Sequenz, wie z.B. zur Anpassung an den exprimierenden Organismus/Zelltyp (Codonnutzung), werden ebenso ermöglicht wie Veränderungen der Sequenz zur Optimierung funktioneller genetischer Parameter, wie beispielsweise Genregulation.

Ebenfalls ermöglicht werden Veränderungen der Sequenz zur Optimierung funktioneller Parameter des Transkripts, z.B. Spleißen, Regulation auf mRNA-Ebene, Regulation auf Translationsebene, und darüber hinaus die Optimierung funktioneller Parameter des Genprodukts, wie z.B. die Aminosäuresequenz (z.B. Antikörper, Wachstumsfaktoren, Rezeptoren, Kanäle, Poren, Transporter etc.).

Darüber hinaus ist es möglich, Konstrukte zu erstellen, die über den RNAi-Mechanismus in die Genexpression eingreifen. Wenn solche Konstrukte für mehr als eine RNAi-Spezies kodieren, können in einem Multiplex-Ansatz mehrere Gene gleichzeitig inhibiert werden.

Insgesamt ist das mit dem Verfahren realisierte System extrem flexibel und erlaubt in bisher nicht vorhandener Weise die programmierte Erstellung von genetischem Material mit stark verringertem Aufwand an Zeit, Materialien und Arbeit.

Größere DNA-Moleküle, wie z.B. Chromosomen von mehreren hundert kbp, waren mit den vorhandenen Methoden nahezu nicht gezielt manipulierbar. Bereits komplexere (d.h. größere) virale Genome von mehr als 30 kbp (z.B. Adenoviren) sind mit den klassischen Methoden der Gentechnik schwierig zu handhaben und zu manipulieren.

Es kommt zu einer erhebliche Verkürzung bis zur letzten Stufe der Klonierung eines Gens: Das Gen oder die Gene werden als DNA-Molekül synthetisiert und dann (nach geeigneter Vorbereitung, wie Reinigung etc.) direkt in Zielzellen eingebracht und das Ergebnis studiert. Der mehrstufige, meist über Mikroorganismen, wie E.coli, laufende Klonierungsprozess (z.B. DNA-Isolation, Reinigung, Analyse, Rekombination, Klonierung in Bakterien, Isolation, Analyse etc.) wird damit auf die letzte Übertragung des DNA-Moleküls in die finalen Effektorzellen reduziert. Bei synthetisch hergestellten Genen oder Genfragmenten ist eine klonale Vermehrung in einem Zwischenwirt (zumeist E.coli) nicht mehr notwendig. Damit umgeht man die Gefahr, dass das für die Zielzelle bestimmte Genprodukt eine toxische Wirkung auf den Zwischenwirt ausübt. Damit hebt man sich deutlich ab von der Toxizität mancher Genprodukte, die bei der Verwendung klassischer Plasmid-Vektoren häufig zu erheblichen Problemen bei der Klonierung der entsprechenden Nukleinsäurefragmente führt.

Eine weitere erhebliche Verbesserung ist die Zeitverkürzung und die Reduktion der Arbeitsschritte, bis nach dem Sequenzieren von genetischem Material die dabei vorgefundenen potentiellen Gene als solche verifiziert und kloniert werden. Normalerweise werden nach Auffinden interessierender Muster, die als ORF in Frage kommen, mit Sonden (z.B. mittels PCR) in cDNA-Bibliotheken entsprechende Klone gesucht, die allerdings nicht die ganze Sequenz der ursprünglich bei ihrer Herstellung verwendeten Boten-RNA (messenger RNA, mRNA) enthalten müssen (Problem der "full lenght clones"). Bei anderen Verfahren wird mittels eines Antikörpers in einer Expressions-Genbibliothek gesucht (Screening). Beide Verfahren lassen sich mit dem erfahrungsgemäßen Verfahren extrem abkürzen: bei Vorliegen einer "in silico" bestimmten Gen-Sequenz (d.h. nach der Erkennung eines entsprechenden Musters in einer DNA Sequenz durch den Computer), oder nach Entschlüsselung einer Proteinsequenz, kann ein entsprechender Vektor mit der Sequenz oder Varianten davon direkt über programmierte Synthese eines integrierten genetischen Elementes erzeugt und in geeignete Zielzellen eingebracht werden.

Die so erfolgende Synthese von DNA-Molekülen bis zu mehreren 100 kbp erlaubt die direkte Komplett-Synthese von viralen Genomen, z.B. Adenoviren. Diese sind ein wichtiges Werkzeug in der Grundlagenforschung (u.a. Gentherapie), aber wegen der Größe ihres Genoms (ca. 40 kbp) schwer mit klassischen Methoden der Gentechnik zu handhaben. Besonders die rasche und ökonomische Erzeugung von Varianten zur Optimierung ist dadurch stark limitiert. Diese Limitierung wird durch das erfindungsgemäße Verfahren aufgehoben.

Durch das Verfahren erfolgen die Integration der Synthese, die Ablösung der Syntheseprodukte und die Zusammenlagerung zu einem DNA-Molekül in einem System. Mit Herstellungsverfahren der Mikrosystemtechnik können alle notwendigen Funktionen und Verfahrensschritte bis zur Aufreinigung des finalen Produktes in einem miniaturisierten Reaktionsträger integriert werden. Dies können Synthesebereiche, Ablösungsbereiche (Cluster), Reaktionsräume, Zuführungskanäle, Ventile, Pumpen, Konzentratoren, Auftrennungsbereiche etc. sein.

Plasmide und Expressionsvektoren können für sequenzierte Proteine oder entsprechende Teilsequenzen direkt hergestellt und die Produkte biochemisch und funktionell analysiert werden, z.B. unter Verwendung geeigneter Regulationselemente. Damit fällt die Suche nach Klonen in einer Gen-Bibliothek weg. Entsprechend können offene Leseraster ("open reading frames" ORF) aus Sequenzierarbeiten (z.B. Humangenomprojekt) direkt in entsprechende Vektoren programmiert und mit gewünschten genetischen Elementen kombiniert werden. Eine Identifikation von Klonen, z.B. in durch aufwendiges Screening von cDNA Bibliotheken, entfällt. Damit ist der Informationsfluss von der Sequenz-Analyse zur Funktions-Analyse stark verkürzt worden, denn am selben Tag, an dem ein ORF durch Analyse von Primärdaten im Computer vorliegt, kann ein entsprechender Vektor inklusive des vermuteten Gens synthetisiert und zur Verfügung gestellt werden.

Gegenüber konventioneller Festphasen-Synthese zur Gewinnung synthetischer DNA zeichnet sich das Verfahren gemäß Erfindung durch geringeren Materialaufwand aus. Zur Herstellung tausender von unterschiedlichen Bausteinen für die Erzeugung von einem komplexen integrierten genetischen Element mit mehreren 100.000 kbp Länge, in entsprechend parallelisiertem Format und bei entsprechender Miniaturisierung (siehe Ausführungsbeispiele), braucht ein mikrofluidisches System deutlich weniger Einsatzstoffe als ein konventioneller Festphasensynthese-Automat für einen einzelnen DNA-Oligomer (bei Verwendung einer einzigen Säule). Hier stehen Mikroliter dem Verbrauch von Millilitern gegenüber, d.h. ein Faktor von 1000.

Unter Berücksichtigung neuester Erkenntnisse der Immunologie erlaubt das vorgestellte Verfahren ein extrem rationelles und schnelles Impfstoff-Design (DNA-Vakzine).

### 1.5.9 Kompetitionsassays mit Gemisch aus Nukleinsäure-Sonden an fester Phase und Lösung

Es kann eine Kompetition von festphasenimmobilisierten Sonden und kurzen Nukleinsäuren in Lösung um Bindung an Target-Nukleinsäuren durchgeführt werden.

### 1.5.10 Starke Bevorzugung von Volllängen-Nukleinsäuren auf dem Array als Kopiermatrizen

Prinzipiell kann der enzymatische Kopiervorgang distal, proximal oder entlang der festphasenimmobilisierten Nukleinsäurepolymeren initiiert werden. Sollte er distal gestartet werden, so ergibt sich ein Zusatzaspekt: das Verfahren kopiert dann im Wesentlichen nur Volllängenprodukte und umgeht so das potentielle Problem von Abbruchprodukten aus der *in situ* Synthese auf dem Reaktionsträger, die dann keine Vervielfältigung erfahren und somit nicht in der Population der Kopien in ihrer umgeschriebenen Form enthalten sind.

### 1.5.11 Verbesserung des Anteils an Volllängen-Nukleinsäuren auf dem Array durch Rückreaktion

Durch Auffüllen verkürzter, aber korrekter Sonden durch Rückreaktion der Kopien von Volllängenprodukten kann der Anteil an Volllängen-Nukleinsäuren erhöht werden.

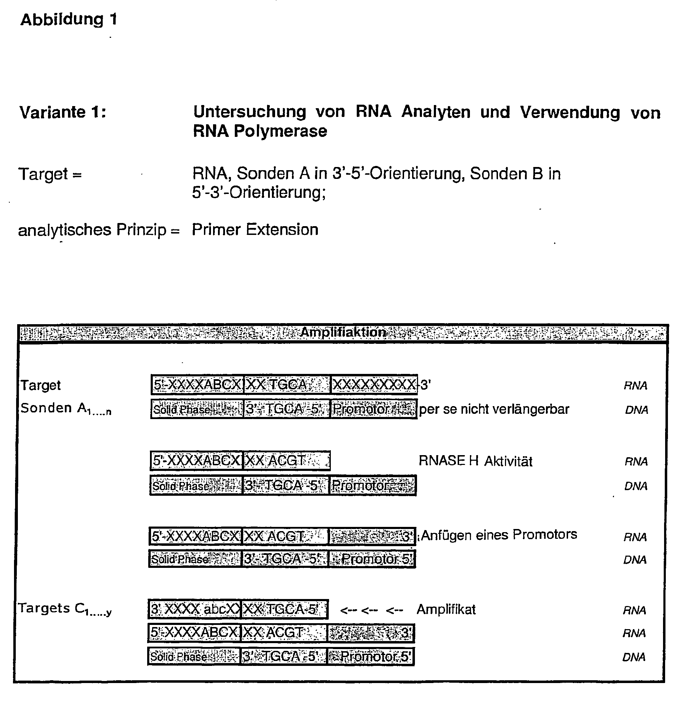

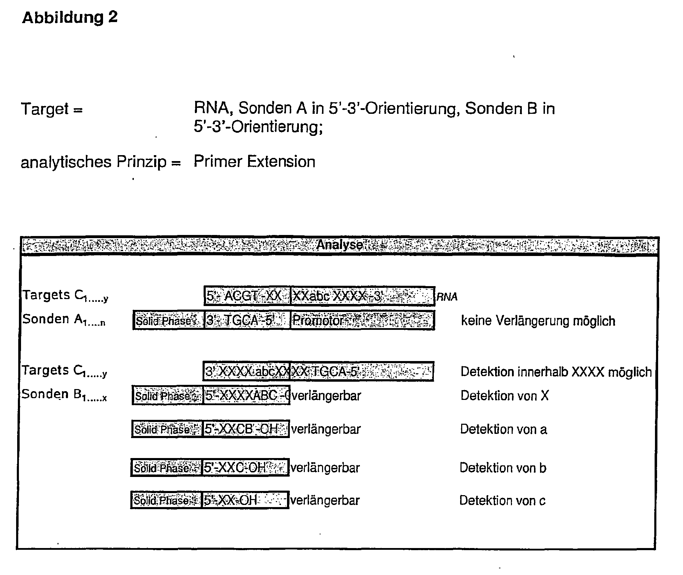

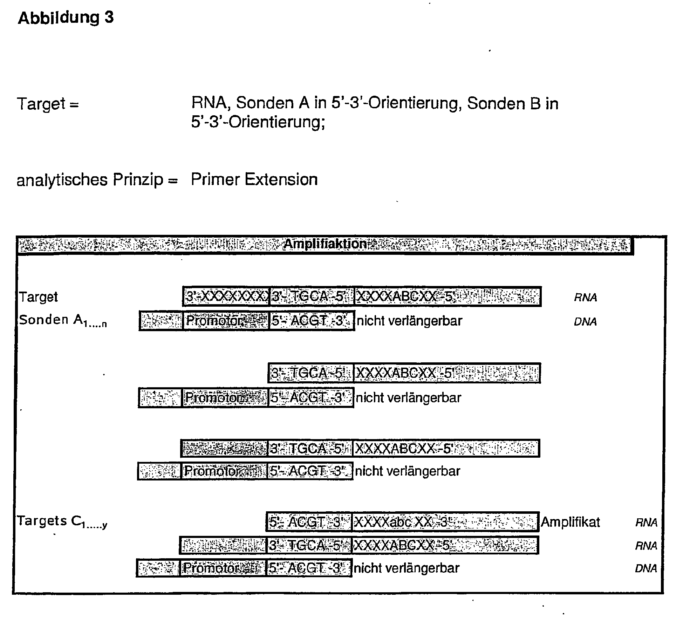

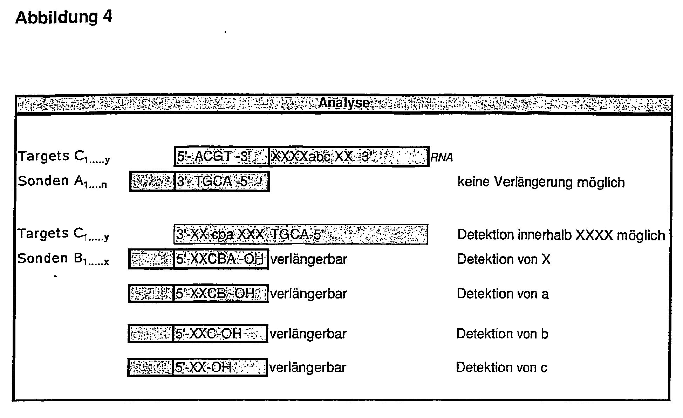

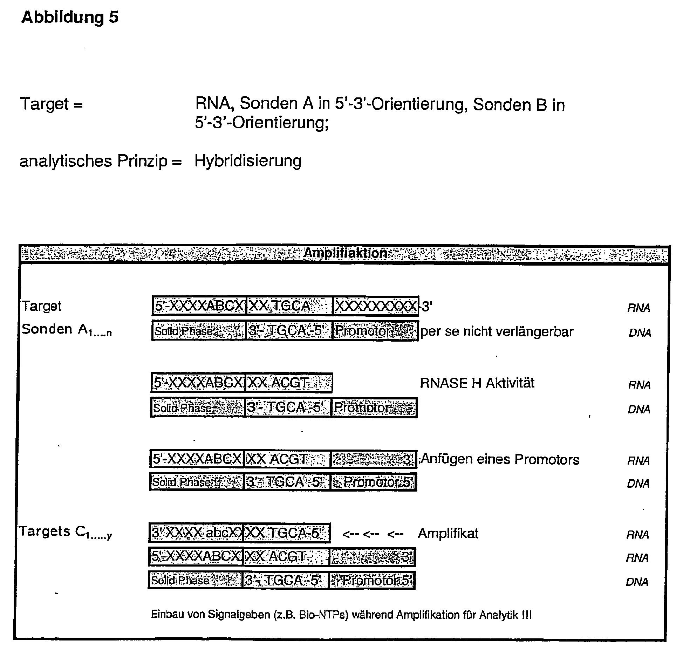

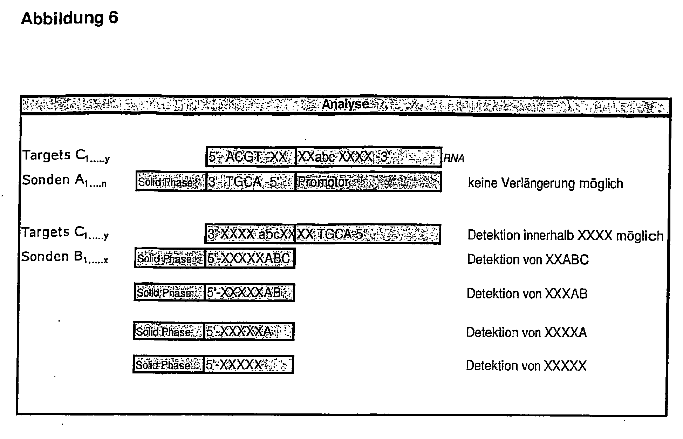

## Patentansprüche

1. Verfahren zur Herstellung einer Mehrzahl von unterschiedlichen synthetischen Nukleinsäuren, umfassend die Schritte:
(a) Bereitstellen eines Trägers mit einer Oberfläche, die eine Vielzahl von Positionen enthält, an denen jeweils unterschiedliche Nukleinsäurefragmente vorhanden sind, umfassend Basensequenzen, die komplementär zu den herzustellenden Nukleinsäuren sind,
(b) Zuführen von Nukleotidbausteinen und eines Enzyms, das ein Erzeugen von unterschiedlichen Nukleinsäuren aus den komplementären Basensequenzen aus (a) bewirkt und
(c) Ablösen der in Schritt (b) erzeugten Nukleinsäuren,
wobei die Nukleinsäurefragmente aus (a) so gewählt werden, dass sich die in Schritt (b) gebildeten Nukleinsäuren bzw. Teilsequenzen zu Nukleinsäuredoppelstrang-Hybriden zusammenlagern können.

2. Verfahren zur Herstellung eines Nukleinsäuredoppelstrangs, umfassend die Schritte:
(a) Bereitstellen eines Trägers mit einer Oberfläche, die eine Vielzahl von Positionen enthält, an denen jeweils unterschiedliche Nukleinsäurefragmente vorhanden sind, umfassend Basensequenzen, die komplementär zu Teilsequenzen des herzustellenden Nukleinsäuredoppelstrangs sind,
(b) Zuführen von Nukleotidbausteinen und eines Enzyms, das ein Erzeugen von Teilsequenzen des herzustellenden Nukleinsäuredoppelstrangs aus den komplementären Basensequenzen aus (a) bewirkt, und
(c) Assemblieren der in Schritt (b) erzeugten Teilsequenzen zu dem gewünschten Nukleinsäurestrang,
wobei die Nukleinsäurefragmente aus (a) so gewählt werden,
dass sich die in Schritt (b) gebildeten Nukleinsäuren bzw. Teilsequenzen zu Nukleinsäuredoppelstrang-Hybriden zusammenlagern können.

3. Verfahren nach einem der Ansprüche 1 oder 2,
**dadurch gekennzeichnet**,
dass der Träger ausgewählt wird aus flachen Trägern, porösen Trägern, Reaktionsträgern mit Elektroden, Reaktionsträgern mit Partikeln oder Beads, mikrofluidischen Reaktionsträgern, die gegebenenfalls Oberflächenmodifikationen aufweisen und Kombinationen der zuvor genannten Träger.

4. Verfahren nach einem der Ansprüche 1 bis 3,
**dadurch gekennzeichnet**,
dass man einen mikrofluidischen Träger bereitstellt.

5. Verfahren nach einem der Ansprüche 1 bis 4,
**dadurch gekennzeichnet**,
dass die Nukleinsäurefragmente aus (a) durch ortsaufgelöste *in situ* Synthese auf dem Träger erzeugt werden.

6. Verfahren nach Anspruch 5,
**dadurch gekennzeichnet**,
dass die Nukleinsäurefragmente aus (a) durch orts- oder/und zeitaufgelöste Belichtung mittels einer programmierbaren Lichtquellenmatrix synthetisiert werden.

7. Verfahren nach Anspruch 6,
**dadurch gekennzeichnet**,
dass die orts- oder/und zeitaufgelöste Synthese in einem mikrofluidischen Träger mit einem oder mehreren fluidischen Reaktionsräumen und einem oder mehreren Reaktionsbereichen innerhalb eines fluidischen Reaktionsraumes erfolgt.

8. Verfahren nach einem der Ansprüche 2 bis 7,
**dadurch gekennzeichnet**,
dass das Assemblieren der Teilsequenzen gemäß Schritt (c) zumindest teilweise in einem oder mehreren Schritten auf dem Träger erfolgt.

9. Verfahren nach einem der Ansprüche 1 bis 8,
**dadurch gekennzeichnet**,
dass mehrere Nukleinsäuren bzw. Teilsequenzen, die einen Strang des Nukleinsäuredoppelstrangs bilden, kovalent miteinander verbunden werden.

10. Verfahren nach Anspruch 9,
**dadurch gekennzeichnet**,
dass das kovalente Verbinden eine Behandlung mit Ligase oder/und ein Auffüllen von Lücken in den Strängen mit DNA-Polymerase umfasst.

11. Verfahren nach einem der Ansprüche 1 bis 10,
**dadurch gekennzeichnet**,
dass Schritt (b) das Zuführen von mindestens einem Primer pro Position des Trägers umfasst, wobei der Primer komplementär zu einem Teil des an dieser Position befindlichen Nukleinsäure-Fragments ist und Schritt (b) eine Elongation des Primers umfasst.

12. Verfahren nach einem der Ansprüche 1 bis 10,
**dadurch gekennzeichnet**,
dass in Schritt (a) doppelsträngige Nukleinsäurefragmente bereitgestellt werden, wobei mindestens ein Strang an der Oberfläche des Träger verankert wird.

13. Verfahren nach Anspruch 12,
**dadurch gekennzeichnet**,
dass Schritt (b) eine Transkription von doppelsträngigen DNA-Fragmenten oder/und eine Replikation von doppelsträngigen RNA-Fragmenten umfasst.

14. Verfahren nach einem der Ansprüche 1 bis 10,
**dadurch gekennzeichnet**,
dass in Schritt (a) Nukleinsäurefragmente bereitgestellt werden, umfassend ein selbstprimendes 3'-Ende und Schritt (b) eine Elongation des 3'-Endes umfasst.

15. Verfahren nach Anspruch 14, das eine Abspaltung des Elongationsproduktes umfasst.

16. Verfahren nach einem der Ansprüche 1 bis 15,
**dadurch gekennzeichnet**,
dass in Schritt (a) doppelsträngige, ringförmige Nukleinsäurefragmente bereitgestellt werden, wobei ein Strang an der Oberfläche des Trägers verankert wird und der andere Strang ein selbstprimendes 3'-Ende umfasst und Schritt (b) eine Elongation des 3'-Endes umfasst.

17. Verfahren nach Anspruch 16, das eine Abspaltung des Elongationsprodukts umfasst.

18. Verfahren nach einem der Ansprüche 1 bis 17,
**dadurch gekennzeichnet**,
dass die Nukleinsäurefragmente aus (a) erzeugt werden durch:
- Bereitstellen von Fängersonden an den Positionen und
- Binden von Nukleinsäurefragmenten aus einem über den
Träger geleiteten Fluid an die Fängersonden,
wobei die Fängersonden komplementär zu Teilbereichen der Nukleinsäurefragmente sind.

19. Verfahren nach einem der Ansprüche 1 bis 18,
**dadurch gekennzeichnet**,
dass in der Sequenz der Nukleinsäure bzw. des Nukleinsäuredoppelstrangs an einer oder mehreren Positionen Erkennungssequenzen für die spezifische Interaktion mit Molekülen enthalten sind.

20. Verfahren nach einem der Ansprüche 1 bis 19,
**dadurch gekennzeichnet**,
dass die Sequenz der Nukleinsäure bzw. der Nukleinsäuredoppelstränge eine natürlich vorkommende Sequenz, eine nicht natürlich vorkommende Sequenz oder eine Kombination aus diesen beiden ist.

21. Verfahren nach einem der Ansprüche 1 bis 20,
**dadurch gekennzeichnet**,
dass die Sequenz aus einer Datenbank, einem Sequenzierexperiment oder einer Vorrichtung zur integrierten Synthese und Analyse von Polymeren entnommen ist.

22. Verfahren nach einem der Ansprüche 1 bis 21,
**dadurch gekennzeichnet**,
dass die Nukleotidbausteine in der Natur vorkommende Nukleotide, modifizierte Nukleotide oder Mischungen davon enthalten können.

23. Verfahren nach einem der Ansprüche 1 bis 22,
**dadurch gekennzeichnet**,
dass modifizierte Nukleotidbausteine zur Markierung und späteren Detektion der Nukleinsäuren bzw. der zusammengelagerten Nukleinsäuredoppelstränge verwendet werden.

24. Verfahren nach Anspruch 23,
**dadurch gekennzeichnet**,
dass als Markierungsgruppen lichtabhängig oder/und elektrochemisch zu detektierende Moleküle verwendet werden.

25. Verfahren nach einem der Ansprüche 1-24, weiterhin umfassend die Herstellung einer Zusammensetzung für therapeutische oder pharmakologische Zwecke, welche die Nukleinsäure beziehungsweise den Nukleinsäuredoppelstrang umfasst.

26. Verfahren nach einem der Ansprüche 1-24, weiterhin umfassend die Herstellung einer Zusammensetzung für diagnostische Zwecke, welche die hergestellten Nukleinsäuren beziehungsweise den hergestellten Nukleinsäuredoppelstrang enthält.

27. Verfahren nach einem der Ansprüche 25-26, umfassend eine Umsetzung in Effektorzellen.

28. Verfahren nach einem der Ansprüche 1-24, wobei die hergestellten Nukleinsäuren bzw. Nukleinsäuredoppelstränge während einer stufenweisen Kombination und Zusammenlagerung oder im Anschluss daran stabilisiert, kondensiert oder/und topologisch manipuliert werden.

29. Verfahren nach Anspruch 28,
wobei die Stabilisierung, Kondensation oder/und topologische Manipulation durch funktionelle Moleküle erfolgt.

30. Verfahren nach einem der Ansprüche 1 bis 24 und 28 und 29, wobei die hergestellten Nukleinsäuren bzw. Nukleinsäuredoppelsträngen als propagierbarer Klonierungs-Vektor in geeigneten Zielzellen der Transkription, der Expression der transkribierten Sequenz oder der Gewinnung von exprimierten Genprodukten dienen kann.

## Claims

1. A method for preparing a plurality of different synthetic nucleic acids, comprising the steps:
(a) provision of a support with a surface which comprises a plurality of positions at each of which different nucleic acid fragments are present, comprising base sequences which are complementary to the nucleic acids to be prepared,
(b) addition of nucleotide building blocks and of an enzyme which brings about generation of different nucleic acids from the complementary base sequences from (a), and
(c) detachment of the nucleic acids generated in step (b),
wherein the nucleic acid fragments from (a) are chosen so that the nucleic acids or partial sequences formed in step (b) can be joined to give nucleic acid double-stranded hybrids.

2. A method for preparing a nucleic acid double strand, comprising the steps:
(a) provision of a support with a surface which comprises a plurality of positions at each of which different nucleic acid fragments are present, comprising base sequences which are complementary to partial sequences of the nucleic acid double strand to be prepared,
(b) addition of nucleotide building blocks and of an enzyme which brings about generation of partial sequences of the nucleic acid double strand to be prepared from the complementary base sequences from (a), and
(c) assembly of the partial sequences generated in step (b) to give the
desired nucleic acid strand,
wherein the nucleic acid fragments from (a) are chosen so that the nucleic acids or partial sequences formed in step (b) can be joined to give nucleic acid double-stranded hybrids.

3. The method as claimed in claim 1 or 2,
**characterized in that**
the support is selected from flat supports, porous supports, reaction supports with electrodes, reaction supports with particles or beads, microfluidic reaction supports which optionally have surface modifications, and combinations of the aforementioned supports.

4. The method as claimed in any of claims 1 to 3,
**characterized in that**
a microfluidic support is provided.

5. The method as claimed in any of claims 1 to 4,
**characterized in that**
the nucleic acid fragments from (a) are generated by spatially resolved *in situ* synthesis on the support.

6. The method as claimed in claim 5,
**characterized in that**
the nucleic acid fragments from (a) are synthesized by spatially or/and time-resolved illumination by means of a programmable light source matrix.

7. The method as claimed in claim 6,
**characterized in that**
the spatially or/and time-resolved synthesis takes place in a microfluidic support with one or more fluidic reaction chambers and one or more reaction zones within a fluidic reaction chamber.

8. The method as claimed in any of claims 2 to 7,
**characterized in that**
the assembly of the partial sequences in step (c) takes place at least partly in one or more steps on the support.

9. The method as claimed in any of claims 1 to 8,
**characterized in that**
a plurality of nucleic acids or partial sequences which form a strand of the nucleic acid double strand are covalently connected together.

10. The method as claimed in claim 9,
**characterized in that**
the covalent connection comprises a treatment with ligase or/and a filling-in of gaps in the strands with DNA polymerase.

11. The method as claimed in any of claims 1 to 10,
**characterized in that**
step (b) comprises the addition of at least one primer for each position of the support, the primer being complementary to part of the nucleic acid fragment located at this position, and step (b) comprising an elongation of the primer.

12. The method as claimed in any of claims 1 to 10,
**characterized in that**
double-stranded nucleic acid fragments are provided in step (a), with at least one strand being tethered to the surface of the support.

13. The method as claimed in claim 12,
**characterized in that**
step (b) comprises transcription of double-stranded DNA fragments or/and replication of double-stranded RNA fragments.

14. The method as claimed in any of claims 1 to 10,
**characterized in that**
nucleic acid fragments comprising a self-priming 3' end are provided in step (a), and step (b) comprises elongation of the 3' end.

15. The method as claimed in claim 14, which comprises elimination of the elongation product.

16. The method as claimed in any of claims 1 to 15,
**characterized in that**
double-stranded, circular nucleic acid fragments are provided in step (a), one strand being tethered to the surface of the support, and the other strand comprising a self-priming 3' end, and step (b) comprising elongation of the 3' end.

17. The method as claimed in claim 16, which comprises elimination of the elongation product.

18. The method as claimed in any of claims 1 to 17,
**characterized in that**
the nucleic acid fragments from (a) are generated by:
- provision of capture probes at the positions and
- binding of nucleic acid fragments from a fluid
passed over the support to the capture probes,
wherein the capture probes are complementary to partial regions of the nucleic acid fragments.

19. The method as claimed in any of claims 1 to 18,
**characterized in that**
recognition sequences for specific interaction with molecules are present at one or more positions in the sequence of the nucleic acid or of the nucleic acid double strand.

20. The method as claimed in any of claims 1 to 19,
**characterized in that**
the sequence of the nucleic acid or of the nucleic acid double strands is a naturally occurring sequence, a non-naturally occuring sequence or a combination of these two.

21. The method as claimed in any of claims 1 to 20,
**characterized in that**
the sequence is taken from a database, from a sequencing experiment or from an apparatus for integrated synthesis and analysis of polymers.

22. The method as claimed in any of claims 1 to 21,
**characterized in that**
the nucleotide building blocks may comprise naturally occurring nucleotides, modified nucleotides or mixtures thereof.

23. The method as claimed in any of claims 1 to 22,
**characterized in that**
modified nucleotide building blocks are used for labeling and subsequent detection of the nucleic acids or of the joined nucleic acid double strands.

24. The method as claimed in claim 23,
**characterized in that**
molecules to be detected in a light-dependent or/and electrochemical manner are used as labeling groups.

25. The method as claimed in any of claims 1-24, further comprising the production of a composition comprising the nucleic acid or nucleic acid double strand, for therapeutic or pharmacological purposes.

26. The method as claimed in any of claims 1-24, further comprising the
production of a composition comprising the nucleic acids prepared or the nucleic acid double strand prepared, for diagnostic purposes.

27. The method as claimed in any of claims 25-26, comprising a transfer into
effector cells.

28. The method as claimed in any of claims 1-24, wherein the nucleic acids or
nucleic acid double strands prepared are stabilized, condensed or/and topologically manipulated during a stepwise combination and joining or subsequent thereto.

29. The method as claimed in claim 28,
wherein the stabilization, condensation or/and topological manipulation is effected by functional molecules.

30. The method as claimed in any of claims 1 to 24 and 28 and 29, wherein the nucleic acids or nucleic acid double strands prepared can serve as propagatable cloning vector in suitable target cells for transcription, for expression of the transcribed sequence, and for the isolation of expressed gene products.

## Revendications

1. Procédé de préparation d'un certain nombre d'acides nucléiques synthétiques différents, comprenant les étapes de :
(a) préparation d'un support avec une surface qui comprend un certain nombre de positions, sur lesquelles des fragments d'acides nucléiques différents sont chaque fois présents, comprenant des séquences de bases, qui sont complémentaires des acides nucléiques à préparer,
(b) addition d'éléments nucléotides et d'une enzyme, qui cause la production d'acides nucléiques différents à partir des séquences de bases complémentaires de (a), et
(c) séparation des acides nucléiques produits à l'étape (b),
où les fragments d'acides nucléiques de (a) sont choisis de sorte que les acides nucléiques et respectivement les séquences partielles formés à l'étape (b) peuvent s'associer en hybrides d'acides nucléiques double brin.

2. Procédé de préparation d'un double brin d'acide nucléique, comprenant les étapes de :
(a) préparation d'un support avec une surface qui comprend un certain nombre de positions, sur lesquelles des fragments d'acides nucléiques différents sont chaque fois présents, comprenant des séquences de bases, qui sont complémentaires de séquences partielles du double brin d'acide nucléique à préparer,
(b) addition d'éléments nucléotides et d'une enzyme, qui cause la production de séquences partielles du double brin d'acide nucléique à préparer à partir des séquences de bases complémentaires de (a), et
(c) assemblage des séquences partielles produites à l'étape (b) en le brin d'acide nucléique souhaité,
où les fragments d'acides nucléiques de (a) sont choisis de sorte que les acides nucléiques et respectivement les séquences partielles formés à l'étape (b) peuvent s'associer en hybrides d'acides nucléiques double brin.

3. Procédé selon l'une des revendications 1 ou 2, **caractérisé en ce que** le support est choisi parmi des supports plats, des supports poreux, des supports réactionnels avec des électrodes, des supports réactionnels avec particules ou billes, des supports réactionnels microfluidiques, qui présentent le cas échéant, des modifications de surface, et des combinaisons des supports cités ci-dessus.

4. Procédé selon l'une des revendications 1 à 3, **caractérisé en ce que** l'on prépare un support microfluidique.

5. Procédé selon l'une des revendications 1 à 4, **caractérisé en ce que** les fragments d'acides nucléiques de (a) sont produits par synthèse *in situ* de manière spatialement sélective, sur le support.

6. Procédé selon la revendication 5, **caractérisé en ce que** les fragments d'acides nucléiques de (a) sont synthétisés par illumination spatialement et/ou temporellement sélective à l'aide d'une matrice programmable de source lumineuse.

7. Procédé selon la revendication 6, **caractérisé en ce que** la synthèse spatialement et/ou temporellement sélective est réalisée dans un support microfluidique présentant un ou plusieurs espaces de réaction fluide et une ou plusieurs zones de réaction au sein d'un espace de réaction fluide.

8. Procédé selon l'une des revendications 2 à 7, **caractérisé en ce que** l'assemblage des séquences partielles selon l'étape (c) est réalisé au moins partiellement sur le support, en une ou plusieurs étapes.

9. Procédé selon l'une des revendications 1 à 8, **caractérisé en ce que** les acides nucléiques et respectivement, séquences partielles, qui forment un brin de l'acide nucléique double brin, sont liés l'un à l'autre de manière covalente.

10. Procédé selon la revendication 9, **caractérisé en ce que** la liaison covalente comprend un traitement avec une ligase et/ou le remplissage de lacunes dans les brins avec une ADN polymérase.

11. Procédé selon l'une des revendications 1 à 10, **caractérisé en ce que** l'étape (b) comprend l'addition d'au moins une amorce par position du support, où l'amorce est complémentaire d'une partie du fragment d'acide nucléique se trouvant en cette position et l'étape (b) comprend un allongement de l'amorce.

12. Procédé selon l'une des revendications 1 à 10, **caractérisé en ce que** l'on prépare à l'étape (a), des fragments d'acide nucléique double brin, où au moins un brin est ancré sur la surface du support.

13. Procédé selon la revendication 12, **caractérisé en ce que** l'étape (b) comprend la transcription de fragments d'ADN double brin et/ou la réplication de fragments d'ARN double brin.

14. Procédé selon l'une des revendications 1 à 10, **caractérisé en ce que** l'on prépare à l'étape (a), des fragments d'acides nucléiques, qui comprennent une extrémité 3' auto-amorçante et l'étape (b) comprend un allongement de l'extrémité 3'.

15. Procédé selon la revendication 14, qui comprend le clivage des produits d'allongement.

16. Procédé selon l'une des revendications 1 à 15, **caractérisé en ce que** l'on prépare à l'étape (a), des fragments d'acides nucléiques annulaires, double brin, où un brin est ancré sur la surface du support et l'autre brin comprend une extrémité 3' auto-amorçante, et l'étape (b) comprend l'allongement de l'extrémité 3'.

17. Procédé selon la revendication 16, qui comprend le clivage des produits d'allongement.

18. Procédé selon l'une des revendications 1 à 17, **caractérisé en ce que** les fragments d'acides nucléiques de (a) sont produits par :
- préparation de sondes de capture sur les positions, et
- liaisons aux sondes de capture, de fragments d'acides nucléiques depuis un fluide passé sur le support,
où les sondes de capture sont complémentaires à des zones partielles des fragments d'acides nucléiques.

19. Procédé selon l'une des revendications 1 à 18, **caractérisé en ce que** sont présentes dans la séquence de l'acide nucléique et respectivement, de l'acide nucléique double brin, en une ou plusieurs positions, des séquences de reconnaissance pour l'interaction spécifique avec des molécules.

20. Procédé selon l'une des revendications 1 à 19, **caractérisé en ce que** la séquence de l'acide nucléique et respectivement, de l'acide nucléique double brin, est une séquence naturelle, une séquence non naturelle ou une combinaison de celles-ci.

21. Procédé selon l'une des revendications 1 à 20, **caractérisé en ce que** la séquence est prélevée dans une banque de données, une expérience de séquençage ou un dispositif pour la synthèse intégrée et l'analyse de polymères.

22. Procédé selon l'une des revendications 1 à 21, **caractérisé en ce que** les éléments nucléotides peuvent contenir des nucléotides naturels, des nucléotides modifiés ou leurs mélanges.

23. Procédé selon l'une des revendications 1 à 22, **caractérisé en ce que** les éléments nucléotides modifiés sont utilisés pour le marquage et la détection ultérieure de l'acide nucléique et respectivement, de l'acide nucléique double brin associé.

24. Procédé selon la revendication 23, **caractérisé en ce que** l'on utilise comme groupes de marquage, des molécules détectées par la lumière et/ou de manière électrochimique.

25. Procédé selon l'une des revendications 1 à 24, comprenant en outre, la préparation d'une composition pour usages thérapeutiques ou pharmacologiques, qui comprend l'acide nucléique et respectivement, l'acide nucléique double brin.

26. Procédé selon l'une des revendications 1 à 24, comprenant en outre, la préparation d'une composition pour usages diagnostiques, qui contient l'acide nucléique préparé et respectivement, l'acide nucléique double brin préparé.

27. Procédé selon l'une des revendications 25 et 26, comprenant une transformation dans des cellules effectrices.

28. Procédé selon l'une des revendications 1 à 24, où les acides nucléiques et respectivement, acides nucléiques double brin préparés sont stabilisés, condensés ou/et manipulés de manière topologique pendant une combinaison et association graduelles ou suite à celles-ci.

29. Procédé selon la revendication 28, où la stabilisation, la condensation ou/et la manipulation topologique est réalisée par des molécules fonctionnelles.

30. Procédé selon l'une des revendications 1 à 24 et 28 et 29, ou l'acide nucléique et respectivement, acide nucléique double brin préparé peut servir à la transcription, l'expression de la séquence transcrite ou l'obtention de produits géniques exprimés en tant que vecteur de clonage propageable dans des cellules cibles appropriées.
